(19) Europäisches Patentamt / European Patent Office / Office européen des brevets

(11) **EP 4 356 829 A2**

(12) # EUROPEAN PATENT APPLICATION

(43) Date of publication:
**24.04.2024 Bulletin 2024/17**

(21) Application number: **24162537.5**

(22) Date of filing: **03.03.2021**

(51) International Patent Classification (IPC):
***A61B 5/11*** *(2006.01)*

(52) Cooperative Patent Classification (CPC):
**A61B 5/7275; A61B 5/0031; A61B 5/024;
A61B 5/0245; A61B 5/1116; A61B 5/686;
A61B 5/746; G16H 10/20; G16H 10/60;
G16H 20/10; G16H 20/30; G16H 20/60;
G16H 40/40; G16H 40/63; G16H 50/20;** (Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.05.2020 US 202063021775 P**

(62) Document number(s) of the earlier application(s) in
accordance with Art. 76 EPC:
**21160444.2 / 3 906 841**

(71) Applicant: **Pacesetter, Inc.
Sylmar, CA 91342 (US)**

(72) Inventors:
• **DAWOUD, Fady
Studio City, 91604 (US)**
• **GILL, Jong
Valencia, 91355 (US)**

(74) Representative: **Barker Brettell Sweden AB
Kungsbroplan 3
112 27 Stockholm (SE)**

Remarks:
This application was filed on 11.03.2024 as a
divisional application to the application mentioned
under INID code 62.

(54) **METHOD AND SYSTEM FOR HEART CONDITION DETECTION USING AN ACCELEROMETER**

(57) A system (100, 1000) for monitoring a physiologic condition of a patient including an accelerometer (101, 1170) configured to be implanted in the patient, the accelerometer (101, 1170) configured to collect multi-dimensional (MD) accelerometer data along at least two axes, memory (1160) configured to store program instructions and one or more processors (1121) that, when executing the program instructions, are configured to initiate a data collection interval in connection with a patient activity, collect the MD accelerometer data during the patient activity for at least one of a select period of time or until receiving a data collection halt instruction, calculate a travel-related (TR) parameter based on the MD accelerometer data, correlate the TR parameter with physiologic data collected during the patient activity and store the TR parameter and the physiologic data as indicators of a current physiologic state of the patient.

FIG. 1

**(Cont. next page)**

EP 4 356 829 A2

(52) Cooperative Patent Classification (CPC): (Cont.)
**G16H 50/30; G16H 50/70;** A61B 5/0205

## Description

BACKGROUND

Embodiments herein generally relate to methods and systems for detecting a health condition of a heart by using an accelerometer implanted within a patient.

Various implantable devices have been proposed that include a three-dimensional (3-D) accelerometer that is configured to detect movement of the patient during day to day activities. For example, an accelerometer may be part of an implantable cardiac monitor (ICM) or within another similar implantable medical device (IMD) to detect rotation based on the position and/or orientation of the ICM.

The 3-D accelerometer is often underutilized. An accelerometer is capable of generating 3 separate accelerometer signals along corresponding X, Y and Z axes. However, typically in use, a determination is made regarding which one of the 3 axes provides the best read out for a given posture. The single "best" axis is used for data collection moving forward. Meanwhile, the other axes simply are not utilized during data collection. This underutilization of the axes of the 3-D accelerometer leads to numerous inefficiencies.

Additionally, physicians may prescribe activity programs for patients used to determine if a potential health condition is present, or worsening. For example, a six-minute walk test is a test where a patient is requested to walk for six-minutes while travel-related (TR) data is taken. The data may include the average speed of the patient, distance traveled, increases and decreases in speed during the six-minutes, or the like. A probability that the patient exhibits certain health conditions may be determined based on the data.

While the six-minute walk test, along with similar tests may be effective at determining or predicting health conditions, improvements are desired. Specifically, the six-minute walk test is administered during a visit to a clinician. Often months may pass before data is collected, analyzed, and a diagnosis is made that the health condition of the patient has changed.

SUMMARY

In accordance with embodiments herein, a system for monitoring a physiologic condition of a patient is provided. The system includes an accelerometer configured to be implanted in the patient, and the accelerometer is configured to collect multi-dimensional (MD) accelerometer data along at least two axes. The system also includes memory configured to store program instructions, and one or more processors that, when executing the program instructions, are configured to collect the MD accelerometer data during the patient activity for at least one of a select period of time or until receiving a data collection halt instruction. The one or more processors are also configured to determine patient activity parameters based on the MD accelerometer data, collect one or more physiologic signals from the patient to determine a physiologic parameter, assign a risk factor threshold for each patient activity parameter and physiologic parameter to form risk weighted parameters, combine the risk weighted parameters to form a risk weighted composite index, and determine a physiologic status of the patient based on the risk weighted composite index.

Optionally, to determine the patient activity parameters the one or more processors are further configured to calculate a travel-related (TR) parameter based on the MD accelerometer data obtained during the patient activity, calculate an activity intensity level based on the MD accelerometer data, and collect posture related date using the MD accelerometer as a function of time. In another aspect, the one or more processors are further configured to combine the accelerometer signals along the at least two axes to form a composite activity signal that is independent of an orientation of the accelerometer. In another example, the one or more processors are further configured to determine whether the risk weighted composite index exceeds a threshold, and identify a treatment diagnosis or treatment notification based on the risk weighted composite index when the risk weighted composite index exceeds the threshold.

Optionally, the risk factor threshold is based on one of sustained decrease in activity duration during a determined interval, sustained decrease in activity intensity during the determined interval, increase of S3 or S3/S1 heart sound signal amplitudes during the determined interval, or increase in sleep or non-active time duration during the determined interval. Alternatively, the risk factor threshold is based on one of increase of heart rate variability during the determined interval, increase of respiration rate or variability during sleep during the determined interval, increase in sleep apnea hypopnea index during the determined interval, increase in chest impedance during the determined interval, or increase in atrial fibrillation during the determined interval. In yet another embodiment, the risk factor threshold is based on one of increase in duration of an incline posture during a determined interval, or increase in duration of a supine position during the determined interval.

Optionally, to determine the risk weighted composite index, the one or more processors are configured to determine a regression risk based on the patient activity parameters and the physiologic parameter. In another aspect, the select period of time is six minutes. In yet another example, the TR parameter is one of distance traveled, average velocity, maximum velocity, minimum velocity, median velocity, average acceleration, heart rate, blood pressure, or blood sugar level. In an example, the MD acceleration data includes one of calibration data, posture related data, or cardiac activity data. In an example, the risk weighted composite index is a total risk score. In an aspect, physiologic data includes one of age, weight, height, body mass index, tobacco use, alcohol use, previously diagnosed health conditions, or previous surgeries.

In accordance with embodiments herein, a computer implemented method for monitoring a physiologic condition of a patient is provided. The method includes collecting multi-dimensional (MD) accelerometer data along at least two axes during a patient activity for at least one of a select period of time or until receiving a data collection halt instruction, and determining patient activity parameters based on the MD accelerometer data. The method also includes collecting one or more physiologic signals from the patient to determine a physiologic parameter, assigning a risk factor threshold for each patient activity parameter and physiologic parameter to form risk weighted parameters, combining the risk weighted parameters to form a risk weighted composite index, and determining a physiologic status of the patient based on the risk weighted composite index.

Optionally, determining the patient activity parameters includes calculating a travel-related (TR) parameter based on the MD accelerometer data obtained during the patient activity, calculating an activity intensity level based on the MD accelerometer data, and collecting posture related date using the MD accelerometer as a function of time. In another aspect, the method also includes combining the accelerometer signals along the at least two axes to form a composite activity signal that is independent of an orientation of the accelerometer. In another example, the method includes determining whether the risk weighted composite index exceeds a threshold, and identifying a treatment diagnosis or treatment notification based on the risk weighted composite index when the risk weighted composite index exceeds the threshold.

Optionally, the risk factor threshold is based on one of sustained decrease in activity duration during a determined interval, sustained decrease in activity intensity during the determined interval, increase of S3 or S3/S1 heart sound signal amplitudes during the determined interval, or increase in sleep or non-active time duration during the determined interval. Alternatively, the risk factor threshold is based on one of increase of heart rate variability during the determined interval, increase of respiration rate or variability during sleep during the determined interval, increase in sleep apnea hypopnea index during the determined interval, increase in chest impedance during the determined interval, or increase in atrial fibrillation during the determined interval. In another example, the risk factor threshold is based on one of increase in duration of an incline posture during a determined interval, or increase in duration of a supine position during the determined interval.

Optionally, determining the risk weighted composite index comprises determining a regression risk based on the patient activity parameters and the physiologic parameter. In another aspect, the select period of time is six minutes. In another aspect, the TR parameter is one of distance traveled, average velocity, maximum velocity, minimum velocity, median velocity, average acceleration, heart rate, blood pressure, or blood sugar level. In one example, the MD acceleration data includes one of calibration data, posture related data, or cardiac activity data. In one aspect, the risk weighted composite index is a total risk score. In another aspect, physiologic data includes one of age, weight, height, body mass index, tobacco use, alcohol use, previously diagnosed health conditions, or previous surgeries.

In accordance with embodiments herein, a system is provided for monitoring a physiologic condition of a patient. The system includes an accelerometer configured to be implanted in the patient, the accelerometer configured to collect multi-dimensional (MD) accelerometer data along at least two axes. The system includes a memory configured to store program instructions, and one or more processors. When executing the program instructions, the one or more processors are configured to initiate a data collection interval in connection with patient activity, collect the MD accelerometer data during the patient activity for at least one of a select period of time or until receiving a data collection halt instruction, and calculate a TR parameter based on the MD accelerometer data. The one or more processors is also configured to correlate the TR parameter with physiologic data collected during the patient activity, and store the TR parameter and physiologic data as indicators of a current physiologic state of the patient.

Optionally, the system may also include an implantable medical device (IMD) that houses the accelerometer, first memory of the memory and at least a first processor of the one or more processors, where the first processor may be configured to collect the MD accelerometer data.

Optionally, the first processor may be configured to implement at least one of the calculating of the TR parameter, the correlation of the TR parameter with the physiologic data, or the storing of TR parameter and physiologic data. In another aspect, the IMD may be further configured to wireless transmit, to an external device, at least one of the MD accelerometer data, the TR parameter, or the correlation of the TR parameter and the physiologic data. In another example, the system may further comprise an external device configured to wirelessly communicate with the IMD, the external device including a second memory of the memory and a second processor of the one or more processors, the second processor configured to implement at least one of the calculating of the TR parameter, the correlation of the TR parameter with the physiologic data, or the storing of TR parameter and physiologic data. In another aspect, the patient activity may represent a prescribed activity program.

Optionally, the TR parameter may represent at least one of a distance traveled or a speed, the one or more processors configured to calculate the TR parameter based on an activity profile for the corresponding patient.

Optionally, the one or more processors may be further configured to develop a motion model by collecting and correlating multiple levels of baseline MD accelerometer data while a patient travels at corresponding baseline speeds. In another aspect, the calculating may include calculating a speed of movement based on the MD accelerometer data and the motion model. In another aspect, the motion model includes activity ranges associated predetermined speeds, the one or more processors further configured to bin the MD accelerometer data into the activity ranges and based thereon determine speeds traveled for sub-intervals of time corresponding to segments of the MD accelerometer data.

In one or more embodiments, a computer implemented method for monitoring a physiologic condition of a patient may be provided that may include utilizing one or more processors for, initiating a data collection interval in connection with patient activity, and collecting multi-dimensional (MD) accelerometer data along at least two axes from an accelerometer implanted in the patient during the patient activity. The one or more processors may also be utilized for calculating a travel-related (TR) parameter based on the MD accelerometer data, correlating the TR parameter with physiologic data collected during the patient activity, and storing the TR parameter and physiologic data as indicators of a current physiologic state of the patient.

Optionally, the initiating, collecting, calculating, correlating and storing may be performed at least one of continuously, periodically, in response to the physiologic data satisfying select criteria or in an on-demand basis in response to a clinician request.

Optionally, the initiating may include conveying an initiating instruction from a local external device to an implantable medical device and generating an instruction from the external device for the patient to begin the activity, tracking the data collection interval with a timer; when the timer times-out, conveying a halt instruction from the external device to the IMD and generating a halt instruction from the external device.

Optionally, the method may also include repeating the collecting and calculating periodically, sorting values for the TR parameter to form an activity histogram indicative of an estimate of patient activity on a periodic basis. In one aspect, the TR parameter may include speed, and the activity histogram represents a period speed histogram, the correlating utilizing a highest speed activity exhibited by the patient during each data collection interval as a basis for the current physiologic state.

Optionally, the method may also include wirelessly communicating between an external device and an implantable medical device (IMD), wherein at least one of the calculating of the TR parameter, the correlation of the TR parameter with the physiologic data, or the storing of TR parameter and physiologic data are implemented at the external device.

Optionally, the patient activity may include implementing a prescribed activity program by a patient. In another aspect, the TR parameter may represent at least one of a distance traveled or a speed, the calculating including calculating TR parameter based on an activity profile histogram.

Optionally, the method may include developing a motion model by collecting and correlating multiple levels of baseline MD accelerometer data while a patient travels at corresponding baseline speed. In another aspect, the motion model may include activity ranges associated predetermined speeds, the method further comprising identifying sub-intervals of time during which the MD accelerometer data is indicative of a common speed of travel or a speed band having a predetermined speed range, and binning the MD accelerometer data into the activity ranges and based thereon determine speeds traveled for the sub-intervals of time corresponding to segments of the MD accelerometer data.

In one or more embodiment a system for monitoring patient posture may be provided, including an accelerometer configured to be implanted in the patient, the accelerometer configured to generate accelerometer signals along at least first and second axes, the accelerometer signals collectively defining a multidimensional (MD) accelerometer data set. The system may also include a memory configured to store program instructions and to store first and second baseline MD accelerometer data sets associated with first and second reference postures, respectively, and one or more processors. When executing the program instructions, the one or more processors may be configured to collect, from the accelerometer, a candidate MD accelerometer data set while in a candidate patient posture, the candidate MD accelerometer data set including candidate accelerometer signals along the at least first and second axes, and calculate a candidate - baseline (CB) posture relation. The CB posture relation is calculated between i) the candidate accelerometer signals from the candidate MD accelerometer data set, ii) the accelerometer signals from the first baseline MD accelerometer data set associated with the first reference posture, and iii) the accelerometer signals from the second baseline MD accelerometer data set associated with the second reference posture. The CB posture relation may be indicative of a relation between the candidate patient posture and the first and second reference postures. The one or more processors may also be configured to define and store an actual patient posture based on the CB posture relation.

Optionally, each of the MD accelerometer data sets may include accelerometer signals collected along X, Y and Z axes that are orthogonal to one another.

Optionally, the CB posture relation may include a first posture relation value indicative of an extent to which the candidate patient posture is common with the first reference posture, and a second posture relation value indicative of an extent to which the candidate patient posture is common with the first reference posture, the one or more processors configured to define the actual patient posture based on the first and second extent values. In one aspect, the first posture relation value is indicative of an extent to which the candidate patient posture corresponds to a vertical posture, and the second posture relation value is indicative of an extent to which the candidate patient posture corresponds to a supine posture. In another aspect, the one or more processors may further be configured to calculate the first and second posture relation values by calculating first and second vector cross products between the accelerometer signals from the candidate MD accelerometer data set and the accelerometer signals from the first and second baseline MD accelerometer data sets.

Optionally, the one or more processors may also be configured to define a threshold map that divides posture relation values into different segments, where each of the segments are indicative of an extent to which a candidate patient posture corresponds to one of the first and second reference postures. In another aspect, the threshold map may include separate segments indicative of an extent to which the candidate patient posture corresponds to at least two of: i) a standing posture, ii) a supine posture, iii) left lateral recumbent posture, iv) right lateral recumbent posture, v) combination of supine and standing postures, vi) combination of supine and left lateral recumbent postures, vii) combination of supine and right lateral recumbent postures, or viii) a recline posture. Optionally, the one or more processors may be further configured to calculate the CB posture relation by calculating: a vector product of at least two vectors of the at least first and second axes.

In one example, the one or more processors may further be configured to collect, from the accelerometer, the candidate accelerometer signals along the first axis during a first interval, and the candidate accelerometer signals along the second axis during a second interval. In another aspect, the first interval does not overlap with the second interval. Alternatively, the second interval does not begin until the candidate accelerometer signals along the first axis exceed a measurement threshold.

In one or more embodiments, a computer implemented method for monitoring a patient posture may be provide that includes utilizing one or more processors for collecting, from the accelerometer, a candidate MD accelerometer data set while in a candidate patient posture, the candidate MD accelerometer data set including candidate accelerometer signals along the at least first and second axes, and calculating a candidate - baseline (CB) posture relation. The CB posture relation may be calculated between: i) the candidate accelerometer signals from the candidate MD accelerometer data set, ii) the accelerometer signals from the first baseline MD accelerometer data set associated with the first reference posture, and iii) the accelerometer signals from the second baseline MD accelerometer data set associated with the second reference posture. The CB posture relation may be indicative of a relation between the candidate patient posture and the first and second reference postures. The one or more processors may also be configured to define and store an actual patient posture based on the CB posture relation.

Optionally, each of the MD accelerometer data sets includes accelerometer signals collected along X, Y and Z axes that are orthogonal to one another.

Optionally, the CB posture relation may include a first posture relation value indicative of an extent to which the candidate patient posture is common with the first reference posture, and a second posture relation value indicative of an extent to which the candidate patient posture is common with the first reference posture, the one or more processors configured to define the actual patient posture based on the first and second extent values. In another aspect, the first posture relation value may be indicative of an extent to which the candidate patient posture corresponds to a vertical posture, and the second posture relation value may be indicative of an extent to which the candidate patient posture corresponds to a supine posture. In yet another aspect, the method may include calculating the first and second posture relation values by calculating first and second vector cross products between the accelerometer signals from the candidate MD accelerometer data set and the accelerometer signals from the first and second baseline MD accelerometer data sets.

Optionally, the method may also include defining a threshold map that divides posture relation values into different segments, where each of the segments are indicative of an extent to which a candidate patient posture corresponds to one of the first and second reference postures. In one aspect, the threshold map includes separate segments indicative of an extent to which the candidate patient posture corresponds to at least two of: i) a standing posture, ii) a supine posture, iii) left lateral recumbent posture, iv) right lateral recumbent posture, v) combination of supine and standing postures, vi) combination of supine and left lateral recumbent postures, vii) combination of supine and right lateral recumbent postures, or viii) a recline posture.

Optionally, the one or more processors may be configured to calculate the CB posture relation by calculating: a vector product of at least two vectors of the at least first and second axes.

Optionally, the method may include collecting, from the accelerometer, the candidate accelerometer signals along the first axis during a first interval, and the candidate accelerometer signals along the second axis during a second interval. In one aspect, the first interval does not overlap with the second interval. In another aspect, the second interval does not begin until the candidate accelerometer signals along the first axis exceed a measurement threshold.

In one or more embodiments, a system for monitoring real-time activity may be provided. The system may include an accelerometer configured to be implanted in the patient, and the accelerometer may be configured to generate accelerometer signals along three axes. The system may also include a memory configured to store program instructions, and one or more processors. When executing the program instructions, the one or more processors may be configured to collect, from the accelerometer, accelerometer signals along the three axes while a patient undergoes activity, combine the accelerometer signals along the three axes to form a composite activity signal that is independent of an orientation of the accelerometer, and define at least one of an activity intensity or activity tolerance based on the composite activity signal.

Optionally, the system may also include an implantable medical device (IMD) that houses the accelerometer, a first memory of the memory, and at least a first processor of the one or more processors. The first processor may be configured to collect the accelerometer signals and form the composite activity signal.

Optionally, each of the composite activity signals may be formed from accelerometer signals collected along X, Y and Z axes that are orthogonal to one another. In another aspect, the accelerometer signals along the three axes may be collected sequentially, over a first interval, a second interval, and a third interval, wherein the first interval, the second interval, and the third interval do not overlap. In another aspect, the one or more processors may be further configured to estimate a patient velocity based on the composite activity signal and a piecewise linear function. In another example, the one or more processors may be further configured to determine a patient distance traveled based on the patient velocity determined. Alternatively, the one or more processors may be further configured to determine a number of steps of a patient from the composite activity signal to define the activity intensity or the activity tolerance. In one aspect, the number of steps of the patient may be based on an amplitude or frequency of the composite activity signal.

Optionally, the one or more processors may be further configured to determine step length of a patient or step duration of a patient from the composite activity signal to define the activity intensity of the activity tolerance. In another aspect, the one or more processors are further configured to filter the composite activity signal to remove activity signals having an amplitude below a threshold amplitude.

In one or more embodiments, a computer implemented method for monitoring real-time activity may be provided and include utilizing one or more processors. The one or more processors may be utilized for collecting, from an accelerometer, accelerometer signals along the three axes while a patient undergoes activity, combining the accelerometer signals along the three axes to form a composite activity signal that is independent of an orientation of the accelerometer, and defining at least one of an activity intensity or activity tolerance based on the composite activity signal.

Optionally, a first processor of the one or more processors collects the accelerometer signals and forms the composite activity signal.

Optionally, the method may also include forming the composite activity signals from accelerometer signals collected along X, Y and Z axes that are orthogonal to one another.

Optionally, the method may also include collecting the accelerometer signals along the three axes sequentially over a first interval, a second interval, and a third interval, wherein the first interval, the second interval, and the third interval do not overlap.

Optionally, the method may also include estimating a patient velocity based on the composite activity signal and a piecewise linear function.

Optionally, the method may also include determining a patient distance traveled based on the patient velocity determined.

Optionally, the method may also include determining a number of steps of a patient from the composite activity signal to define the activity intensity or the activity tolerance. In one aspect, the number of steps of the patient may be based on an amplitude or frequency of the composite activity signal.

Optionally, the method may also include determining step length of a patient or step duration of a patient from the composite activity signal to define the activity intensity of the activity tolerance.

Optionally, the method may also include filtering the composite activity signal to remove activity signals having an amplitude below a threshold amplitude.

BRIEF DESCRIPTION OF THE DRAWINGS

Figure 1 illustrates a block diagram of an accelerometer formed in accordance with embodiments herein.
Figure 2 illustrates a flow block diagram of a method of monitoring a physiologic condition of a patient in accordance with embodiments herein.
Figure 3A illustrates a flow block diagram of a process of calibrating an accelerometer in accordance with embodiments herein.
Figure 3B illustrates a schematic coordinate system of a patient in accordance with embodiments herein.
Figure 3C illustrates a schematic coordinate system of a patient in accordance with embodiments herein.
Figure 3D illustrates a graph of activity at low sampling (dashed e.g. 1 Hz) and higher sampling (solid e.g. 100Hz) on a first multi-dimension axis over time in accordance with embodiments herein.
Figure 3E illustrates a graph of activity on a second multi-dimension axis over time in accordance with embodiments herein.

Figure 3F illustrates a graph of activity on a third multi-dimension axis over time in accordance with embodiments herein.

Figure 3G illustrates a graph of posture as low sampling (dashed e.g. 1 Hz) and higher sampling (solid e.g. 100 Hz) on a first multi-dimension axis over time in accordance with embodiments herein.

Figure 3H illustrates a graph of posture on a second multi-dimension axis over time in accordance with embodiments herein.

Figure 3I illustrates a graph of posture on a third multi-dimension axis over time in accordance with embodiments herein.

Figure 4A illustrates a flow block diagram of a process of monitoring patient posture in accordance with embodiments herein.

Figure 4B illustrates a graph for measuring and predicting patient position in accordance with embodiments herein.

Figure 4C illustrates a graph for measuring patient activity in accordance with embodiments herein.

Figure 5A illustrates a flow block diagram for predicting patient posture using calibration information in accordance with embodiments herein.

Figure 5B illustrates a graph for predicting patient velocity from activity measurements using a piecewise linear function in accordance with embodiments herein.

Figure 6A illustrates a graph for measuring patient activity steps in accordance with embodiments herein.

Figure 6B illustrates a schematic diagram for an accelerometer measuring system to estimate activity steps using operation of low pass filtering and down-sampling in accordance with embodiments herein.

Figure 6C illustrates a graph for measuring patient activity steps in accordance with embodiments herein.

Figure 7 illustrates a graph for measuring patient activity, velocity and distance travelled in accordance with embodiments herein.

Figure 8 illustrates a flow block diagram of a process of monitoring the physiologic condition of a patient based on activity in accordance with embodiments herein.

Figure 9A illustrates a graph of velocity versus accelerometer activity data in accordance with embodiments herein.

Figure 9B illustrates a histogram of velocity during a 24 hour period in accordance with embodiments herein.

Figure 10 illustrates an implantable cardiac monitoring device (ICM) intended for subcutaneous implantation at a site near the heart in accordance with embodiments herein.

Figure 11 illustrates a block diagram of the ICM formed in accordance with embodiments herein.

Figure 12 illustrates a schematic diagram of a healthcare system in accordance with embodiments herein.

Figure 13 illustrates a schematic diagram of a healthcare system with health risk-based alert function in accordance with embodiments herein.

Figure 14 illustrates a schematic diagram of a healthcare system in accordance with embodiments herein.

Figures 15 illustrates a process block diagram of a method for diagnosing heart conditions based on accelerometer data in accordance with embodiments herein.

DETAILED DESCRIPTION

The term "ASM" shall mean application-specific model.

The terms "beat" and "cardiac event" are used interchangeably and refer to both normal and/or abnormal events.

The terms "cardiac activity signal", "cardiac activity signals", "CA signal" and "CA signals" (collectively "CA signals") are used interchangeably throughout to refer to an analog or digital electrical signal recorded by two or more electrodes positioned subcutaneous or cutaneous, where the electrical signals are indicative of cardiac electrical activity. The cardiac activity may be normal/healthy or abnormal/arrhythmic. Non-limiting examples of CA signals include ECG signals collected by cutaneous electrodes, and EGM signals collected by subcutaneous electrodes.

The term "health care system" and "digital health care system" are used interchangeably throughout to reference to a system that includes equipment for measuring health parameters, and communication pathways from the equipment to secondary devices. The secondary devices may be at the same location as the equipment, or remote from the equipment at a different location. The communication pathways may be wired, wireless, over the air, cellular, in the cloud, etc. In one example, the healthcare system provided may be one of the systems described in U.S. Pat. App. 2021/0020294 entitled METHODS DEVICE AND SYSTEMS FOR HOLISTIC INTEGRATED HEALTHCARE PATIENT MANAGEMENT. Other patents that describe example monitoring systems include U.S. Pat. No. 6,572,557; entitled SYSTEM AND METHOD FOR MONITORING PROGRESSION OF CARDIAC DISEASE STATE USING PHYSIOLOGIC SENSORS; U.S. Pat. No. 6,480,733 entitled METHOD FOR MONITORING HEART FAILURE; U.S. Pat. No. 7,272,443 entitled SYSTEM AND METHOD FOR PREDICTING A HEART CONDITION BASED ON IMPEDANCE VALUES USING AN IMPLANTABLE MEDICAL DEVICE; U.S. Pat. No. 7,308,309 entitled DIAGNOSING CARDIAC HEALTH UTILIZING PARAMETER TREND ANALYSIS; and U.S. Pat. No. 6,645,153 entitled SYSTEM AND METHOD FOR EVALUATING RISK OF MORTALITY DUE TO CONGESTIVE HEART FAILURE USING PHYSIOLOGIC SENSORS.

Additionally or alternatively, embodiments herein may be implemented in connection with the methods and systems described in "METHOD AND DEVICE FOR DETECTING RESPIRATION ANOMALY FROM LOW FREQUENCY COMPONENT OF ELECTRICAL CARDIAC ACTIVITY SIGNALS", U.S. Application No. 16/869,733.

Additionally or alternatively, embodiments herein may be implemented in connection with the methods and systems described in "SYSTEM FOR VERIFYING A PATHOLOGIC EPISODE USING AN ACCELEROMETER", U.S. Provisional Application Nos. 63/021,778 and 63/139,304.

The term "IMD" shall mean an implantable medical device. Embodiments may be implemented in connection with one or more implantable medical devices (IMDs). Non-limiting examples of IMDs include one or more of neurostimulator devices, implantable leadless monitoring and/or therapy devices, and/or alternative implantable medical devices. For example, the IMD may represent a cardiac monitoring device, pacemaker, cardioverter, cardiac rhythm management device, defibrillator, neurostimulator, leadless monitoring device, leadless pacemaker and the like. The IMD may measure electrical and/or mechanical information. For example, the IMD may include one or more structural and/or functional aspects of the device(s) described in U.S. Patent Number 9,333,351, entitled "NEUROSTIMULATION METHOD AND SYSTEM TO TREAT APNEA" and U.S. Patent Number 9,044,610, entitled "SYSTEM AND METHODS FOR PROVIDING A DISTRIBUTED VIRTUAL STIMULATION CATHODE FOR USE WITH AN IMPLANTABLE NEUROSTIMULATION SYSTEM". The IMD may monitor transthoracic impedance, such as implemented by the CorVue algorithm offered by St. Jude Medical. Additionally or alternatively, the IMD may include one or more structural and/or functional aspects of the device(s) described in U.S. Patent Number 9,216,285, entitled "LEADLESS IMPLANTABLE MEDICAL DEVICE HAVING REMOVABLE AND FIXED COMPONENTS" and U.S. Patent Number 8,831,747, entitled "LEADLESS NEUROSTIMULATION DEVICE AND METHOD INCLUDING THE SAME". Additionally or alternatively, the IMD may include one or more structural and/or functional aspects of the device(s) described in U.S. Patent Number 8,391,980, entitled "METHOD AND SYSTEM FOR IDENTIFYING A POTENTIAL LEAD FAILURE IN AN IMPLANTABLE MEDICAL DEVICE" and U.S. Patent Number 9,232,485, entitled "SYSTEM AND METHOD FOR SELECTIVELY COMMUNICATING WITH AN IMPLANTABLE MEDICAL DEVICE". Additionally or alternatively, the IMD may be a subcutaneous IMD that includes one or more structural and/or functional aspects of the device(s) described in U.S. Patent no. 10,765,860, entitled "SUBCUTANEOUS IMPLANTATION MEDICAL DEVICE WITH MULTIPLE PARASTERNAL-ANTERIOR ELECTRODES"; U.S. Patent No. 10,722,704, entitled "IMPLANTABLE MEDICAL SYSTEMS AND METHODS INCLUDING PULSE GENERATORS AND LEADS"; U.S. Patent Application 2019/0336747, entitled "SINGLE SITE IMPLANTATION METHODS FOR MEDICAL DEVICES HAVING MULTIPLE LEADS". Further, one or more combinations of IMDs may be utilized from the above described patents and applications in accordance with embodiments herein. Embodiments may be implemented in connection with one or more subcutaneous implantable medical devices (S-IMDs). For example, the S-IMD may include one or more structural and/or functional aspects of the device(s) described in U.S. Patent No. 10,722,704, entitled "IMPLANTABLE MEDICAL SYSTEMS AND METHODS INCLUDING PULSE GENERATORS AND LEADS; U.S. Patent no. 10,765,860, entitled "SUBCUTANEOUS IMPLANTATION MEDICAL DEVICE WITH MULTIPLE PARASTERNAL-ANTERIOR ELECTRODES. The IMD may represent a passive device that utilizes an external power source and/or an active device that includes an internal power source. The IMD may deliver some type of therapy/treatment, provide mechanical circulatory support and/or merely monitor one or more physiologic characteristics of interest (e.g., PAP, CA signals, impedance, heart sounds). Additionally or alternatively, embodiments may be implemented in connection with one or more passive IMDS (PIMDs). Non-limiting examples of PIMDs may include passive wireless sensors used by themselves, or incorporated into or used in conjunction with other IMDs, such as cardiac monitoring devices, pacemakers, cardioverters, cardiac rhythm management devices, defibrillators, neurostimulators, leadless monitoring devices, leadless pacemakers, replacement valves, shunts, grafts, drug elution devices, blood glucose monitoring systems, orthopedic implants, and the like. For example, embodiments may implement one or more structural and/or functional aspects of the device(s) described in U.S. Patent No. 9,265,428 entitled "Implantable Wireless Sensor", U.S. Patent No. 8,278,941 entitled "Strain Monitoring System and Apparatus", U.S. Patent No. 8,026,729 entitled "System and Apparatus for In-Vivo Assessment of Relative Position of an Implant", U.S. Patent No. 8,870,787 entitled "Ventricular Shunt System and Method", and U.S. Patent No. 9,653,926 entitled "Physical Property Sensor with Active Electronic Circuit and Wireless Power and Data Transmission".

The term "obtains" and "obtaining", as used in connection with data, signals, information and the like, include at least one of i) accessing memory of an external device or remote server where the data, signals, information, etc. are stored, ii) receiving the data, signals, information, etc. over a wireless communications link between the ICM and a local external device, and/or iii) receiving the data, signals, information, etc. at a remote server over a network connection. The obtaining operation, when from the perspective of an ICM, may include sensing new signals in real time, and/or accessing memory to read stored data, signals, information, etc. from memory within the ICM. The obtaining operation, when from the perspective of a local external device, includes receiving the data, signals, information, etc. at a transceiver of the local external device where the data, signals, information, etc. are transmitted from an IMD and/or a remote server. The obtaining operation may be from the perspective of a remote server, such as when receiving the data, signals, information, etc. at a network interface from a local external device and/or directly from an IMD. The remote server may also obtain the data, signals, information, etc. from local memory and/or from other memory, such as within a cloud storage environment and/or from the memory of a workstation or clinician external programmer.

The terms "patient data entry device" and "PDE device" shall mean an electronic device that includes a user interface that is configured 1) to receive patient data that is entered by the patient and/or 2) to receive patient data in connection with actions/decisions by the patient. A PDE device is different from an IMD and an accelerometer. The PDE device is configured to receive behavior related medical data that differs from IMD data and that differs from accelerometer data. The PDE devices may include, but are not limited to, smart phones, desktop or laptop computers, tablet devices, smart TVs, fixed cameras, smart watch, wearable heart rate monitor, portable or handheld cameras, recording devices, digital personal assistant (DPA) devices and the like. One nonlimiting example of a PDE device is a smart phone implementing the "HEMAAPP" application, developed at the University of Washington, where the application is configured to utilize a camera within a smart phone to monitor a patient's hemoglobin level (while holding a finger over the camera) and to detect when the patient is in an anemic or other undesirable state related to hemoglobin levels. Another example is a smart phone application developed by Wilbur Lam at the Aflac Cancer and Blood Disorders Center of Children's Healthcare of Atlanta, and Wallace Coulter, a faculty member in the Department of biomedical engineering at Georgia Tech. The PDE device may include an electronic device sold under the trademark ALEXA® by Amazon.com Inc., and/or an electronic device sold under the trademark NOW® by Google LLC., and the like. In addition, the PDE devices may represent various types of devices configured to record audio and/or voice signatures, detect gestures and movements and the like. The PDE device may include a graphical user interface, through which the patient or another user enters the patient data. Optionally, the PDE device may include audio and/or video sensors/cameras that may receive patient data. For example, a user may use a keyboard, touch screen and/or mouse to enter patient data. Optionally, the user may enter the patient data through spoken words (e.g., "Alexa I just took my medication", "Alexa I am eating 3 slices of peperoni pizza", "Alexa I am eating an apple", "Alexa I am drinking a 12 oz. soda and eating a candy bar). Optionally, the PDE device may automatically track actions by a patient, such as through the use of cameras to visually watch a patients actions, through the use of microphones to "listen" to a patient's actions, and/or through the use of other types of sensors (e.g., refrigerator or kitchen cabinet door sensor, sensor on a treadmill). For example, a camera may capture video that is processed by a processor utilizing image recognition to identify what a patient is eating/drinking, when the patient eats/drinks, and how much the patient consumed. Optionally, the BRM device may include a position tracking device sold under the trademark FITBIT® by Fitbit Inc. or other types of position tracking devices. The position tracking device may monitor and collect, as BRM data, movement information, such as a number of steps or distance traveled in a select period of time, a rate of speed, a level of exercise and the like. Optionally, the BRM device may monitor and collect, as BRM data, heart rate.

The terms "posture" and "patient posture" refer to posture positions of a patient, including a standing posture, sitting posture, supine posture, prone posture, horizontal side posture (e.g., laying on one's side), 45 degree incline, and the like.

The term "probability" shall mean, not only a determined percentage or numerical value, but also non-numerical values and corresponding indicators. For example, a risk score algorithm may be used to determine a risk range or risk category a patient falls into with the range or risk category illustrated by color, bars, and the like. Such probability may also include morphological comparisons such as comparisons of waveforms in graphs associated with heart related data. In this manner, probability is simply the factoring or use of event related variable(s) to determine the likelihood an event will or will not occur. The probability thus may be represented in numerous manners, including a risk score, a subsequent risk score, a percentage, a bar graph, a range, a color-coded indicator, text indicia providing an indicator text such as "low", "medium", and "high", pictorially, and the like.

The terms "processor," "a processor", "one or more processors" and "the processor" shall mean one or more processors. The one or more processors may be implemented by one, or by a combination of more than one implantable medical device, a wearable device, a local device, a remote device, a server computing device, a network of server computing devices and the like. The one or more processors may be implemented at a common location or at distributed locations. The one or more processors may implement the various operations described herein in a serial or parallel manner, in a shared-resource configuration and the like.

The term "real-time" shall refer to a time period substantially contemporaneous with an event of interest. The term "real-time," when used in connection with obtaining and/or processing data utilizing an IMD, shall refer to processing operations performed substantially contemporaneous with a physiologic event of interest experienced by a patient. By way of example, in accordance with embodiments herein, physiologic and accelerometer signals are analyzed in real time (e.g., during a cardiac event, while walking/running at a particular speed, during a prescribed physical patient activity or within a few minutes after the cardiac event or prescribed physical patient activity). The term "real-time," when used in connection with a body generated analyte, shall refer to operations performed substantially contemporaneous with an occurrence of a characteristic of interest in a malnutrition state experienced by the patient.

Three-dimensional accelerometer sensors are placed in implantable devices (e.g. pacers, ICD's, ICM's, etc.)

and have the capability to quantify acceleration in the three orthogonal directions (e.g. X, Y, and Z). Typically, only one axis is used to capture activity as a binary true or false state. Provided in this disclosure are methods and processes by which information from the combination of all three axes can be used to quantify subject posture with respect to gravity, and activity. By quantifying activity and posture using all three axes, the acceleration signals may be used for numerous purposes, including determining the velocity or speed of a patient, predicting heart failure status changes, determining exercise tolerance, verifying measurements or treatment diagnosis of other equipment, or the like. For example, in one embodiment, a point mobile application may be provided based on a six minute walking test that uses the accelerometer within an IMD to periodically determine six minute walk test results based on an activity profile histogram.

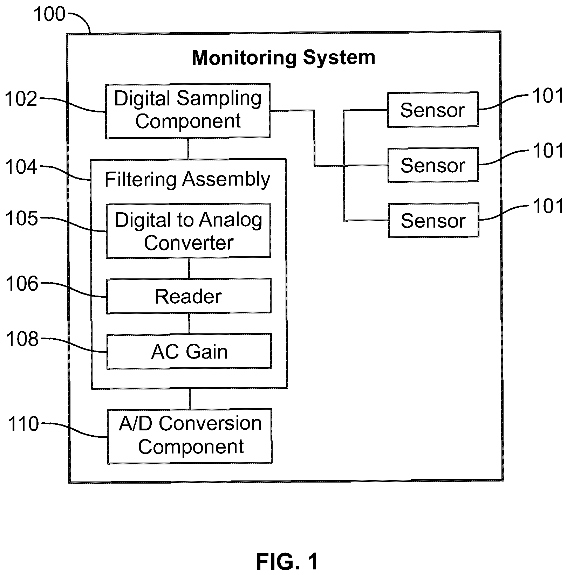

Figure 1 illustrates a schematic diagram of a monitoring system 100. In one example, the monitoring system is or includes an accelerometer. In one embodiment when the monitoring system 100 is an accelerometer, the accelerometer may be a chip for placement in an IMD. In another embodiment, the accelerometer is formed and operates in the manner described in US patent 6,937,900, titled "AC/DC Multi-Axis Accelerometer For Determining A Patient Activity And Body Position". In an embodiment, when the monitoring system is an accelerometer, the accelerometer includes sensors that generate first (X), second (Y) and third (Z) accelerometer signals along corresponding X, Y and Z axes (also referred to as first axis accelerometer signals, second axis accelerometer signals and third axis accelerometer signals). The X, Y and Z axes accelerometer signals collectively define a three-dimensional (3D), or multi-dimensional (MD) accelerometer data set. While examples herein are described in connection with an accelerometer that generates accelerometer signals along three orthogonal axes, it is recognized that embodiments may be implemented wherein accelerometer signals are generated along two or more axes, including more than three axes.

The monitoring system 100 may include sensors 101 that monitor and receive signals from the X, Y and Z axes. In one embodiment, the individual X, Y and Z signals are received by a digital sampling component 102 that receives a digital input. Coupled to the digital sampling component 102 is a filtering assembly 104 that may include a digital to analog converter 105 to form an alternating current (AC) signal, a reader device 106, and an AC gain device 108. While in this embodiment, the filtering assembly includes the devices provided, in other examples, other devices may be utilized to filter the digital input signal for processing.

The monitoring system 100 may also include an analog to digital conversion component 110, along with a position, or direct current (DC) component. In one example, the analog to digital conversion component may be an 8-bit analog to digital converter (ADC). The evaluation version of the monitoring system 100 may provide 3-axis (X and Y along the chip, Z normal to the chip) DC-coupled posture signal corresponding to 3 orthogonal directions as well as 3-axis AC-coupled activity signal. In one embodiment, each of the 6 signal may be sampled at 100 Hz and accumulated over 1 sec for a total of 12 signals([X/Y/Z],[posture/activity],[100/1 Hz]). This MD accelerometer data may be used to describe embodiments herein.

While described as a digital signal in relation to Figure 1, in other embodiments the signal may be an analog signal, filtered, amplified, etc. The accelerometer data signals may be recorded in a data storage of the accelerometer, of an IMD, of a remote device etc. Alternatively, the accelerometer data set may be obtained from a remote device, or received from a storage device coupled to the accelerometer. To this end, the accelerometer data set may be a multi-dimensional accelerometer data set.

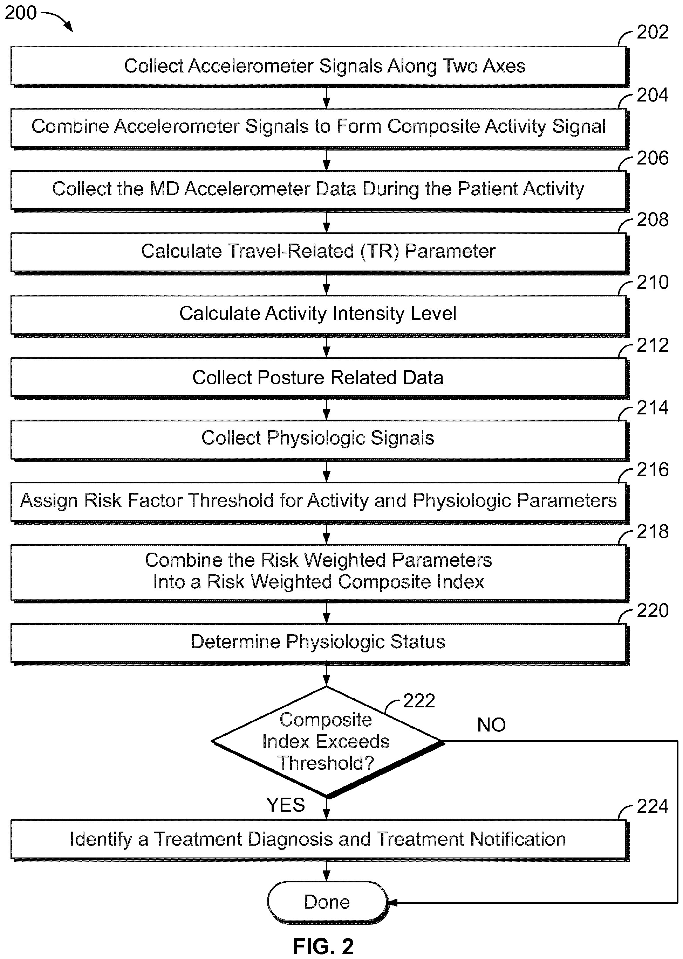

Figure 2 provides an example of how monitoring patient activity with a MD accelerometer may be utilized to provide treatments or alerts related to a patient. Figure 2 illustrates a method 200 for monitoring a physiologic condition of a patient. The method 200 may be implemented at least in part by utilizing the accelerometer described in relation to figure 1.

At 202, one or more processors collect MD accelerometer data along at least two axes during a patient activity for at least one of a select period of time or until receiving a data collection halt instruction. In one example, the MD accelerometer data is collected along each of the X, Y, and Z axes. Alternatively, only two of the three axes are used. Patient activity includes all movements and actions of a patient. Patient activity includes walking, jogging, running, sitting, jumping, sleeping, standing, etc. The patient activity may occur as a result of being requested by a physician. For example, a physician may request a patient perform a six minute walk test where the activity of the patient is monitored while the patient walks for six straight minutes. In another example, the activity may be a change in position or posture of a patient.

At 204, the one or more processors combine the accelerometer signals along the at least two axes to form a composite activity signal that is independent of an orientation of the accelerometer. The composite activity signal may be formed based on a calculation with respect to gravity, based on orthogonal relationships between the axes, or the like. By utilizing the composite activity signal, determination related to accelerometer orientation are eliminated.

At 206, the one or more processors collect the MD accelerometer data during patient activity for at least one of a select period of time or until receiving a halt instruction. The data collection may be initiated by a patient, clinician, automatically based on a schedule, determined condition, or threshold being met, or the like. The data collection interval may occur for a select period of time, until receiving a data collection halt instruction from a patient, clinician, automatically based on a schedule, determined condition, or threshold being met, etc., or the like. A halt instruction may be any signal received by an accelerometer, IMD, PDE, or the like that results in the accelerometer no longer collecting data, and the data collection interval ending.

At 208, one or more processors calculate a travel related (TR) parameter based on the MD accelerometer data collected during the patient activity. TR parameters are patient activity parameters related to travel. TR parameters include any parameter related to the movement or action of a patient. TR parameters may include distance traveled during an interval, average speed or velocity during an interval, maximum speed or velocity during an interval, median speed or velocity during an interval, patient acceleration information, heart rate during travel, blood pressure during travel, blood sugar level during travel, or the like. The TR parameters may be calculated using algorithms, models, mathematical functions, look up tables, decision trees, etc. from the MD accelerometer data provided.

At 210, the one or more processors calculate an activity intensity level from the accelerometer data as a function of time. Activity intensity level is a patient activity parameter related to the intensity of an activity. Intensity is based on the activity of the patient during the period of time. In one example, velocity or distance calculations based on the MD accelerometer data collected during a six minute walk test is utilized in making the calculations. To this end, the activity intensity level may be calculated based on determining the average velocity of the patient during the select period of time, the greatest velocity during the select period of time, the distance traveled during the select period of time, changes in velocity over the select period in time, or the like. The activity intensity level may be calculated using algorithms, models, mathematical functions, look up tables, decision trees, etc. from the MD accelerometer data provided.

At 212, the one or more processors collect posture related data using the MD accelerometer data as a function of time. Similar to TR parameters, and activity intensity level, the posture position is also a patient activity parameter. Posture related data may include data related to different postures, including standing, supine, sitting LLR, RLR, recline etc. The MD accelerometer signals may be collected only in connection with a couple of postures without directly measuring accelerometer signals in connection with other postures. For the postures, for which no accelerometer signals are directly measured, "synthetic" accelerometer signals may be derived for the non-measured postures based on actual accelerometer signals. The calculation of the synthetic accelerometer signals may be implemented utilizing an algorithm, mathematical equation, lookup table, decision tree, or the like.

At 214, one or more processors collect one or more patient physiologic signals from the patient to determine a physiologic parameter. Patient physiologic signals may be utilized to determined patient physiologic parameters that are any health related parameters associated with a patient. Patient physiologic parameters may include patient age, weight, height, body mass index (BMI), tobacco use, alcohol use, previously diagnosed health conditions, previous surgeries, etc. The patient physiologic parameters may be collected from a data storage device, including a data storage device of a remote device such as a physician's computing device, government agency computing device, hospital computing device, in home medical device, IMD, ICM, PDE, etc. The physiologic parameters may also be received from monitoring the patient over time and recording and analyzing collected patient data.

At 216, one or more processors assign a risk factor threshold for each of the TR parameters, and patient activity parameters to form risk weighted parameters. Risk factors are any health related information, including TR parameters, activity intensity level, posture related data, physiologic parameters, etc. that indicated a patient is more likely to experience a pathological episode. Pathologic episodes include heart failure, stroke, syncope, arrythmia, heart attack, asystole, Brady event, neurological episodes, ventricular fibrillation (VF), ventricular tachycardia (VT), a diabetic seizure, and epileptic seizure, or any other type of seizure, episodes that may result from substantial reduction or change in pulmonary arterial pressure and the like.

Risk factors may include a sustained increase in duration of an incline posture over a determined interval, or a sustained increase of heart rate variability over the determined interval. Alternatively, a sustained increase in a S3 or S3/S1 heart sound signal amplitude over the determined interval may also be a risk factor. Other risk factors may be based on heart arrhythmia burden derived from EGM signal, including atrial fibrillation burden, premature ventricular complex burden, or other duration of heart arrhythmia. Other risk factors may be based on sleep, including increase of sleep or non-active time duration over the determined interval; increase of respiration rate or variability during rest or sleep over the determined interval, a sustained increase of sleep apnea hypopnea (AHI) index over the determined interval, or the like. Risk factors may also include a sustained increase in chest impedance over the determined interval, and sustained increase in AF burden over the determined interval.

A risk factor threshold is a weight provided to a risk factor in determining if the pathologic episode is more likely to occur.

Table 1

| Risk factor (RF) value | RF Threshold | RF criteria met? | Allowed RF points | RF severity | RF points = severity*allowed points |
|---|---|---|---|---|---|
| RF1 = 0.1 | 1 | 0.1 >1 ? No | 1 to 2 | na | 0 |
| RF2 = 4 | 2.2 | 4 >2.2 ? Yes | 1 to 3 | 0.3 | 1+0.3*2 = 1.6 |
| RF3 = 3 | 3.5 | 3>3.51 ? -> No | 1 to 4 | na | 0 |
| RF4 = 12 | 9 | 12<1 ? -> Yes | 1 to 5 | 0.9 | 1+0.9*4=4.6 |
| RF2=4 AND RF4=12 | 2.2 , 9 | 4>2.2 AND 12>9-> Yes | 1 to 2 | 0.5 | 1+0.5*1=1.5 |
| Total risk factor score | | | | | 0 + 1.6 + 0 + 4.6 + 1.5 = 7.7 |

As shown in the table, risk factors may be given different weights (e.g. values, points, etc.) based on determined criteria.

At 218, the one or more processors combine the risk weighted parameters into a risk weighted composite index. When combining the risk weighted parameters into a risk weighted composite index (e.g. total risk factor, or total risk score) for a patient, each individual risk factor has a risk factor threshold to be considered triggered. In one example, each triggered risk factor can contribute a limited number of points to an overall risk score as illustrated in the above table based on relative strength to other triggers and based on severity of that specific risk factor (e.g. risk factor 1 can contribute 1 point if its threshold is met and up to 2 additional points depending on severity of this risk factor). A combination of risk factors can also contribute additional points to the overall risk score (e.g. risk weighted composite index) with allowed points/severity as before.

The risk weighted composite index represents a general indicator of a degree to which the patient is experiencing a pathologic episode or potential pathologic episode. As a patient's health deteriorates, indicated by one or more characteristics reflected by the risk weighted parameters, the risk weighted composite index will similarly elevate. In one embodiment, a risk model is provided to determine the risk weighted composite index based on TR parameters, activity intensity level, posture related data, and physiologic data.

In one embodiment, the one or more processors that receive the TR parameter data, activity intensity level, posture related data, and the physiologic data may also communicate with a remote database that includes TR parameter data, activity intensity level, posture related data, and/or physiologic data of other patients. The TR parameter data, activity intensity level, posture related data, and physiologic data of other patients is utilized to determine the risk weighed composite index and/or resulting treatment diagnosis. In one embodiment, an artificial intelligence (AI) algorithm may be utilized that makes a determination based on variables associated with the remote database to determine a determined pathologic episode, or probability of a determined pathologic episode.

For the AI algorithm, weights may be assigned to differing variables related to the TR parameter data, activity intensity level, posture related data, physiologic data, accelerometer data, etc. to make a diagnosis of a pathologic episode such as heart failure. The patient may then undergo an examination to determine if the diagnosis is correct. If the diagnosis is correct, then a reward is provided for the weights providing the correct diagnosis. If the diagnosis is incorrect, no reward is provided, and weights may be varied accordingly.

As an example, if a 70 year-old male lifelong smoker, who is 6 feet tall and 200 lbs, experiences a decrease in activity during a 6 minute walk test compared to a previous test, each variable (e.g. age, smoking habit, height vs. weight, and decrease in average speed) may be given a weight from 1-5 to provide a score regarding diagnosis. The scores may be based on the collected data received from a remote database that has thousands of males in the age group between 68-72, are within 2 inches of 6 foot tall, and 5lbs of 200lbs, smoke at least periodically, and have a similar decrease in activity. Based on the previous diagnosis related to these thousands of males, each variable can then be provided a weight. So, if 80% of the individuals 68-72 that are within 2 inches of 6 foot tall, and 5lbs of 200lbs, smoke at least periodically, and have a similar decrease in activity have heart failure; while heart failure is only seen in 20% of individuals that are 68-72, within 2 inches of 6 foot tall, and 5lbs of 200lbs, and have a similar decrease in activity, but do not smoke at all; the AI algorithm increases the weight provided to smoking for the patient. By using the database, along with an AI algorithm, treatment diagnosis can become more accurate.

With reference back to figure 2, at 220, the one or more processors establish a physiologic status of the patient based on the risk weighted composite index. The physiologic status represents the health of the patient. For example, the physiologic status may be that the patient is in good health, or that the health risks to the patient have not changed over an interval. Alternatively, the physiologic status may be that the patient has a high risk of suffering a heart attack, stroke, or other pathologic episode in the near future. The physiologic status may be established by correlating the risk weighted composite index to similarly situated patients. For example, when the risk weighted composite index is a total risk score, a score between 0-5 may be considered a low risk score based on other patients with similar total risk scores that experienced or did not experience a pathologic episode. Meanwhile a score between 5-10 could indicate a moderate health risk, and any score over 10 could represent a high risk score. The low health risk, moderate heath risk, and high health risk are all examples of a physiologic status. Alternatively, a probability of a pathologic episode or other indicator could represent the physiologic status of the patient.

At 222, the one or more processors make a determination whether the risk weighted composite index exceeds a threshold. In an embodiment when the total risk score is the risk weighted composite index, when a patient scores above a 10 and is within a high risk group, 10 could be considered a threshold score. In this manner, any score 10 or below does not exceed the threshold, while scores above 10 exceed the threshold.

If the risk weighted composite index does not exceed the threshold, flow moves to the right, and no additional action is taken. If the risk weighted composite index does exceed the threshold flow moves downward to 224.

At 224, the one or more processors identify a treatment diagnosis and treatment notification to be provided in connection with the risk weighted composite index. As one example, based on the MD accelerometer signals collected during a six-minute walk test, a change in distance may be determined that increases the risk weighted composite index. As a result, medication dose increase may be recommended as a treatment diagnosis, and an electronic message, or displaying on a display accordingly may also be provided.

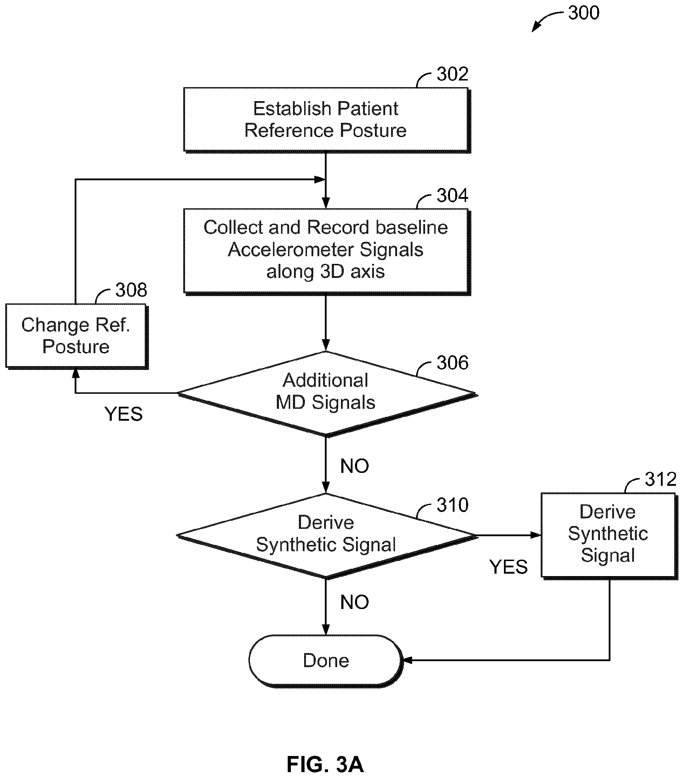

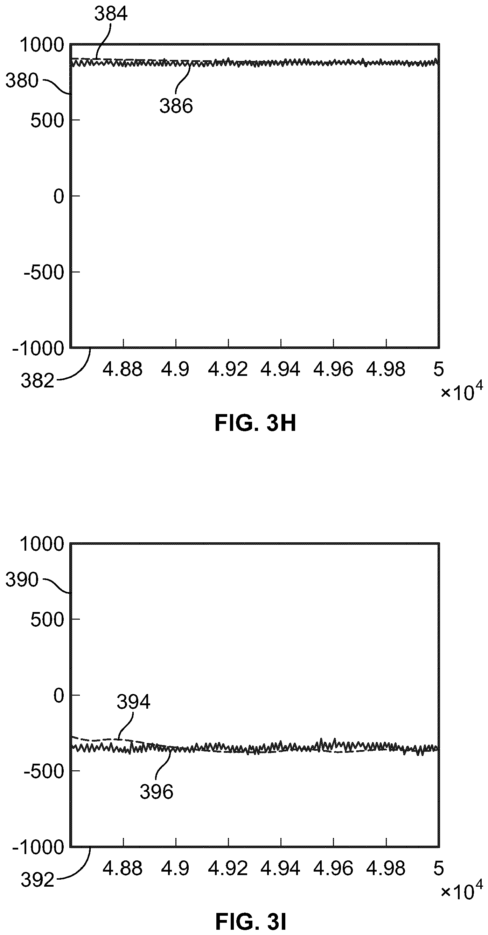

While Figure 1 describes monitoring system, and specifically a 3D accelerometer, and Figure 2 illustrates a general method of using the 3D accelerometer to provide notifications and treatment diagnosis, figure 3A illustrates a method 300 for calibrating the monitoring device. To this end, figure 3A is a block flow diagram of a method 300 of calibration for the accelerometer in accordance with embodiments herein. In one example, the monitoring system is the monitoring system of Figure 1. In one embodiment, the monitoring system may be and ICM, or part of an ICM, that is implanted in a patient. Alternatively, the monitoring system may be included as part of another IMD. The operations of Figure 3A may be implemented at various times, such as when an accelerometer (alone or in combination with a device including the accelerometer) are implanted. Additionally or alternatively, the operations of Figure 3A may be implemented throughout the life of the device, such as periodically, in response to instructions from a local device and/or remote server, in response to various criteria being satisfied (e.g. identification of a change or loss of contact between an ICM and surrounding tissue).

In one embodiment, the calibration may be used to capture the orientation of the monitoring device with respect to gravity and convert the orientation to a human body coordinate system. For example, when a physician implants a device, such as an accelerometer, it is difficult to locate the device in a specific orientation within a patient's anatomy (e.g. aligned parallel to the patient's sternum). As a result, the device may be oriented in various unknown orientations with respect to the patient's anatomy parallel to the sternum, 45° to the sternum, etc. In addition, regardless of the initial orientation of the device with respect to the patient's anatomy, the monitoring device can move within a subcutaneous pocket or other region that receives the implant. The calibration process of Figure 3A captures MD accelerometer data in connection with one or more known body postures to form a baseline MD accelerometer data. As a point hereafter, the baseline MD accelerometer data is then utilized throughout the life of the device as explained herein.

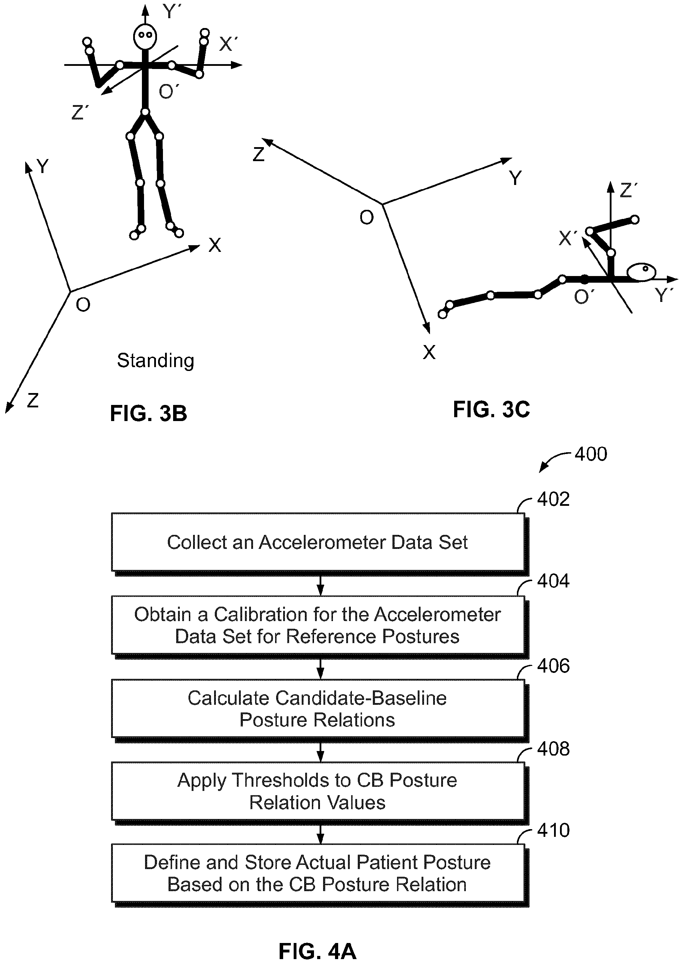

At 302, one or more processors establish a patient reference posture. For example, the patient may be instructed to move to a particular reference posture, such as one of standing, sitting, supine (laying down), left lateral recumbent (LLR), right lateral recumbent (RLR), recline, etc. In one embodiment, as shown in Figure 3B, the patient may be standing such that accelerometer at position O includes an X, Y, and Z axes. Alternatively, the patient may be in a supine position as illustrated in Figure 3C, providing different positions of the X, Y, and Z axes in relation to the O position of the accelerometer.

The instruction may be provided in various manners, such as an instruction from a clinician or other medical personnel who may be actively involved in performing the calibration operations. Additionally or alternatively, the calibration procedure may be implemented without a clinician present, such as through a series of instructions provided to the patient from an application operating on a patient's smart phone or other portable device. The instructions may be part of a preprogrammed calibration process that is triggered based on various criteria. Alternatively, the instructions may be conveyed to the local electronic device (e.g. smart phone, tablet device or otherwise) from a remote server, medical network, physician's computer or otherwise. For example, through telemedicine, a physician may communicate with the patient while being physically remote. Once the patient is in the desired reference posture, the patient may provide an indication, such as speaking or entering an indication through a local device.

At 304, accelerometer sensors obtain accelerometer signals from two or more axes, and the accelerometer signals are recorded as an accelerometer data set. The accelerometer signals may come from at least two of the X-axis, Y-axis, or Z-axis. In one example, the accelerometer signals may be obtained from all three axes.

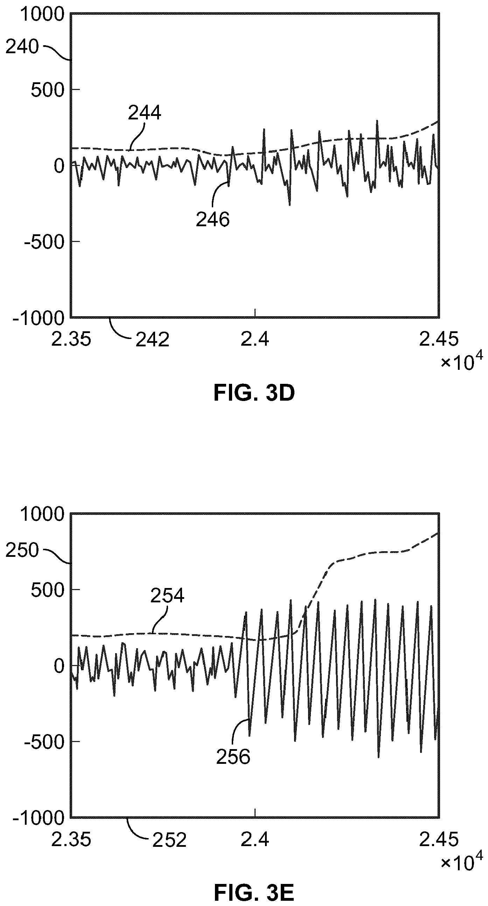

Figures 3D-3I illustrate examples of MD accelerometer signals that may be obtained and recorded over a desired interval (e.g. 1 second). Figures 3D - 3F illustrate activity level along the vertical axis 340, 350, 360 and time along the horizontal axis 342, 352, 362. Figures 3D-3F correspond to the MD accelerometer signals obtained from the X, Y and Z axis channels/outputs, respectively. Figure 3D illustrates an X accelerometer signal 346 obtained from the X axis channel over a period of time 342, where the signal is relatively small during a first period of time and then becomes larger during a second period of time. The X accelerometer signal 346 may be an analog signal that is sampled at a desired rate to become a digital signal (e.g., 100 Hz). In accordance with aspects herein, an average activity 344 may be calculated from the activity level 340. The average activity 344 is relatively small during the first period of time and begins to increase during the second period of time.

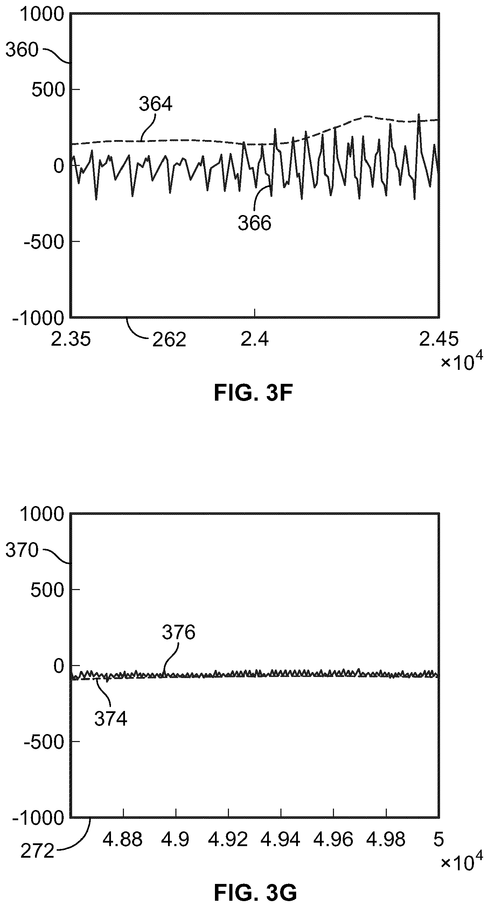

Figures 3E and 3F illustrate Y and Z accelerometer signals 356, 366 obtained from the Y and Z axes channels, respectively, over the same period of time 352, 362. The Y accelerometer signal 356 is initially small (although larger than the initial portion of the X accelerometer signal 346) during a first period of time and then becomes substantially larger (larger than the X accelerometer signal 346) during the second period of time. The Z accelerometer signal 366 maintains a relatively medium amount of activity throughout the measurement period. The Y and Z accelerometer signals 356, 366 may be analog signals that are sampled at a desired rate to become a digital signal (e.g., 100 Hz). In accordance with aspects herein, average activities 354, 364 may be calculated from the Y and Z accelerometer signals 356, 366.

Figures 3G-3I illustrate indications of X, Y and Z posture that are derived from the X, Y and Z accelerometer signals in Figures 3D - 3E, respectively. The vertical axes correspond to a degree or extent of an X, Y, Z posture 370, 380, 390 that is indicated by the corresponding X, Y or Z accelerometer signal. The extent of the X, Y and Z postures range between upper and lower limits corresponding to limits of the corresponding posture (e.g., standing, supine, LLR, RLR, recline, etc.).

The horizontal axes correspond to a common period of time 372, 382, 392 (e.g., 1 second, 30 seconds, 1 minute). The X posture 376 indicates a relatively neutral X posture at 374. The Y posture 386 exhibits a large or strong indication at 384 that the Y posture is near a limit of a Y posture range (e.g., standing). The Z posture 396 indicates a slightly negative Z posture at 374.

As one example, the signals in Figures 3D-3I may be measured and obtained when the patient is standing. The X, Y, and Z accelerometer signals 346, 356, 366 and the X, Y and Z postures 376, 386, 396 may be saved in connection with the standing baseline patient posture.

Returning to Figure 3A, at 306, one or more processors render a decision regarding whether additional MD accelerometer signals should be obtained and recorded in connection with additional postures. If not, the process is complete. In one example, the calibration process may involve obtaining accelerometer signals in connection with numerous postures, such as standing (figure 3B), supine (figure 3C) LLR, RLR, recline, etc.

Alternatively, in some embodiments, accelerometer signals may be obtained only in connection with a couple of postures (also referred to as baseline postures), without directly measuring accelerometer signals in connection with other postures. For the postures, for which no accelerometer signals are directly measured, synthetic accelerometer signals may be derived for the non-measured postures based on actual accelerometer signals measured in connection with the baseline postures. The calculation of the synthetic accelerometer signals may be implemented utilizing an algorithm, mathematical equation, lookup table, decision tree, or the like. When accelerometer signals are to be obtained in connection with additional postures, flow moves to 308. Otherwise, the calibration processes done.

At 308, one or more processors provide instruction for the patient to move to a different posture as compared to the posture related to 302. The instruction may alternatively be provided by a clinician, or be provided electronically by a local external device. Next, flow returns to 304 where new MD accelerometer signals are obtained and a new accelerometer data set is recorded in connection with the new posture. By way of example, a first MD accelerometer data sets may be recorded for a standing position, while a second MD accelerometer data set is recorded in connection with a supine position. Additionally or alternatively, a MD accelerometer data set may be obtained and recorded for an LLR posture at 308. The MD accelerometer data sets may simply be stored and/or may be further analyzed.

Returning to 306, when it is determined that no additional acceleration signals should be measured from individual patient postures, flow moves to 310.

At 310, the one or more processors determine whether to calculate/derive synthetic acceleration signals in connection with one or more additional baseline postures. For example, in accordance with the operations at 302 - 308, the patient may be directed to stand in a standing posture, for which acceleration signals are obtained for a baseline standing posture. When the acceleration signals along the X, Y and Z axes are nonzero, synthetic (e.g. calculated) acceleration signals may be mathematically derived for at least two other baseline postures based on the baseline standing posture, such as for the supine posture and the LLR posture. When it is desirable to calculate acceleration signals for additional baseline postures, flow moves to 312.

At 312, the one or more processors calculate the synthetic acceleration signals in connection with one or more additional baseline postures. The additional baseline postures are orthogonal to a baseline posture for which actual acceleration signals are measured. In the foregoing example, the additional baseline postures for supine and LLR are orthogonal to the standing posture. By way of example, the synthetic acceleration signals may be calculated by the one or more processors utilizing a matrix rotation that applies a 90° angle operation to the acceleration signals obtained in connection with the standing posture (e.g. $A_x$, $A_y$, $A_z$). As a further example, the matrix rotation may be calculated based on the following:

$$ B''' = \begin{bmatrix} 1 & 0 & 0 \\ 0 & 0 & -1 \\ 0 & 1 & 0 \end{bmatrix} \begin{bmatrix} A_X \\ A_Y \\ A_Z \end{bmatrix} = A_X - A_Z + A_Z \text{ and } C''' = \begin{bmatrix} 0 & -1 & 0 \\ 1 & 0 & 0 \\ 0 & 0 & 1 \end{bmatrix} \begin{bmatrix} A_X \\ A_Y \\ A_Z \end{bmatrix} = A_X - A_Z + A_Z $$

In this manner, a measured baseline posture in the standing position is used to derive a synthetic baseline posture that is orthogonal to the measured baseline posture. In one example, the synthetic baseline posture may be the LLR, RLR, recline, or supine posture. In an alternative embodiment, the measured baseline posture may be a supine position, with $B_x$, $B_y$, $B_z$ representing the accelerometer readings along the X axis, Y axis, and Z axis channels while in a supine position, and a matrix rotation, or other mathematical equation, or other function is then used to derive the standing position orthogonal to the supine position. While a mathematical equation, such as a matrix rotation may be used, in other embodiments look up tables, decision trees, functional algorithms, or the like may instead be used to derive the synthetic baseline posture.

Once the baseline postures and corresponding baseline accelerometer signals are determined, the baseline information may be used to monitor patient activity, patient posture, patient health and other information of interest as explained herein.

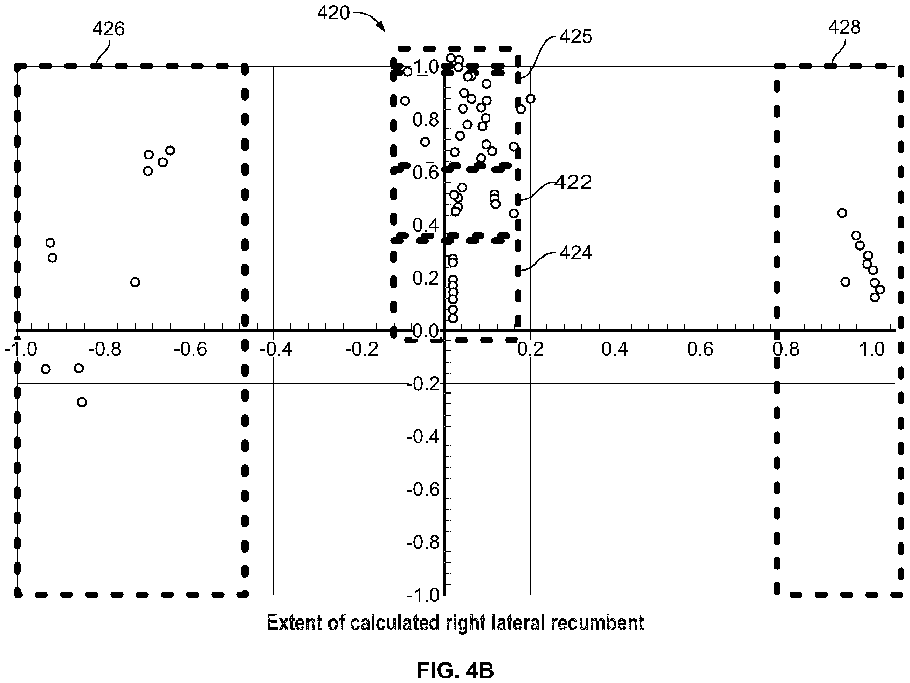

Figure 4A illustrates a method 400 for monitoring posture of a patient using MD accelerometer data in accordance with embodiments herein. Figure 4B illustrates an example graph used to make posture determinations. In one example the monitoring system described in Figure 1 may be used to perform the process. In another example, the posture of the patient may be a patient activity parameter as described in relation to figure 2, or may be used to determine a patient activity parameter. By utilizing all three axes of an accelerometer, the process determines the posture of the patient and changes of the posture of the patient. The accelerometer may transmit MD accelerometer signals continuously and/or transmit one or more MD accelerometer data sets to a PDE device, including a smart phone, desktop or laptop computer, tablet device, smart TV, fixed camera, smart watch, wearable heart rate monitor, portable or handheld camera, recording device, digital personal assistant (DPA) device, and the like.

At 402, an accelerometer data set is obtained while a patient is in a candidate patient posture. To form the accelerometer data set, accelerometer signals are obtained in connection with at least two of the X-axis, the Y-axis, or the Z-axis. The accelerometer data set is referred to as a candidate MD accelerometer data set (e.g. $N_x$, $N_y$, $N_z$) given that the current posture of the patient is unknown at the time that the accelerometer data set is obtained. The candidate patient posture is the posture of the patient in real-time. The candidate MD accelerometer data set may include candidate accelerometer signals along at least first and second axes. In one example, the candidate accelerometer signals accelerometer data may be taken from all three axes.

At 404, one or more processors obtain one or more baseline MD accelerometer data set for two or more reference postures. The baseline MD accelerometer data set may be stored in memory in a monitoring system, IMD, local external device, remote server or otherwise. Additionally or alternatively, the baseline MD accelerometer data set may be wireless transmitted to the monitoring system or IMD from the local external device or remote server. The baseline MD accelerometer data set may include at least first and second MD accelerometer data sets. The first MD accelerometer data set includes accelerometer signals obtained along two or more axes while a patient maintained a first reference posture. The first baseline MD accelerometer data set may be based on measured baseline postures, or synthetic baseline postures. The first reference posture may be considered a candidate - baseline (CB) posture. The CB posture may be any posture of a patient used to compare to other future postures monitored. The second MD accelerometer data set includes accelerometer signals obtained along the two or more axes while the patient maintains a second reference posture. It is recognized that a baseline MD accelerometer data set may include accelerometer signals obtained while a patient maintains various additional postures. Optionally, the first and second MD accelerometer data sets may correspond to postures that are orthogonal to one another (e.g. standing and laying horizontal) and/or that are derived from one another where a first posture is a measured baseline posture and the second posture is a synthetic baseline posture. Additionally or alternatively, the first and second MD accelerometer data sets may correspond to postures that have other known relations to one another.

At 406, the one or more processors calculate candidate-baseline posture relations. The CB posture relations include a first CB posture relation that is calculated between i) the candidate accelerometer signals from the candidate MD accelerometer data set and ii) the accelerometer signals from the first baseline MD accelerometer data set associated with a first reference posture. The CB posture relations include a second CB posture relation that is calculated between i) the candidate accelerometer signals from the candidate MD accelerometer data set and iii) the accelerometer signals from the second baseline MD accelerometer data set associated with the second reference posture. The CB posture relations indicative of a relation/correlation between the candidate patient posture and the first and second reference postures. The calculation may be made by use of an algorithm, mathematical equation, mathematical function, lookup table, decision tree, or the like.

In one embodiment, the first CB posture relation value may be indicative of an extent to which the candidate patient posture is common with the first reference posture, while a second CB posture relation value may be indicative of an extent to which the candidate patient posture is common with the second reference posture. The actual patient posture may then be based on the first and second CB posture relation values. In one example, the first CB posture relation value can be indicative of an extent to which the candidate patient posture corresponds to a vertical posture, and the second CB posture relation value is indicative of an extent to which the candidate patient posture corresponds to a supine posture. Similarly, the first CB posture relation value and second CB posture relation value may be indicative of other positions including standing, LLR, RLR, recline, etc. Additionally, the first and second CB posture relation values may be calculated with first and second vector cross products between the accelerometer signals from the candidate MD accelerometer data set and the accelerometer signals from the first and second baseline MD accelerometer data sets.

By way of example, the MD accelerometer data sets may each be characterized as a vector having a number of dimensions corresponding to the number of axes for which accelerometer signals are obtained. For example, when accelerometer signals are obtained along X, Y and Z axes, the vector would have three dimensions, namely an X dimension, a Y dimension and a Z dimension. The CB posture relation may be calculated as a mathematical function denoting a relation between candidate and baseline vectors. For example, the first baseline MD accelerometer data set may include X, Y and Z accelerometer signals $A_x$, $A_y$, and $A_z$ in connection with a baseline standing posture, while the second baseline MD accelerometer data set may include X, Y and Z accelerometer signals $B_x$, $B_y$, $B_z$ in connection with a baseline supine posture. Optionally, a third baseline MD accelerometer data set may include X, Y and Z accelerometer signals $C_x$, $C_y$, $C_z$ for the LLR posture. As a further option, fourth baseline MD accelerometer data set may include X, Y and Z accelerometer signals $D_x$, $D_y$, $D_z$ for a baseline RLR posture.

The candidate MD accelerometer data set includes X, Y and Z accelerometer signals $N_x$, $N_y$, $N_z$ for an unknown "candidate" current posture. The one or more processors may calculate, as the mathematical relation, vector inner products between the candidate MD accelerometer data set and each of the baseline MD accelerometer data sets. For example, beginning with the standing posture, the one or more processors calculate the vector inner product between the A and N vectors utilizing the following equation:

$$\frac{A_X * N_X + A_Y * N_Y + A_Z * N_Z}{\|A\|\|N\|}$$

The vector inner product represents the extent of correlation between the candidate posture and the baseline standing posture (e.g. in a normalized range 0 to 1). The parameters $\|A\|$ and $\|N\|$ represent the square root of the sum of squared vector components $\sqrt{A_X^2 + A_Y^2 + A_Z^2}$ and $\sqrt{N_X^2 + N_Y^2 + N_Z^2}$.

The vector inner product of the B and N vectors represents the extent of correlation between the candidate posture and the baseline supine posture (-1 to 1). The vector inner product of the C and N vectors represents the extent of correlation between the candidate posture and the baseline LLR posture, and the vector inner product of the D and N vectors represent the extent of correlation between the candidate posture and the baseline RLR posture.

In the foregoing example, it is assumed that the accelerometer data set includes accelerometer signals for three axes. Optionally, the accelerometer data sets may include accelerometer signals for two axes (e.g. X and Y, X and Z, Y and Z). The foregoing calculations may also be implemented in connection with A, B, C, D and N vectors having two elements (two accelerometer signals). As another option, some of the vectors may have only accelerometer signals in connection with two axes, while other vectors may have accelerometer signals in connection with three axes. For example, a candidate MD accelerometer data set may be obtained that includes accelerometer signals along the X, Y and Z axes, but only the X and Y axis accelerometer signals may be utilized to calculate a vector inner product with X and Y axis accelerometer signals for the baseline standing posture, whereas all three of the X, Y and Z axes accelerometer signals from the candidate posture and the baseline sitting posture may be utilized to calculate the vector inner product. Additionally or alternatively, the X and Y axes accelerometer signals may be utilized for calculating a vector inner product for the candidate posture and the baseline standing posture, while the Y and Z axes accelerometer signals may be utilized for calculating a vector inner product for the candidate posture and the baseline supine posture. It is recognized that other combinations of permutations of the X, Y and Z axes accelerometer signals and the various baseline postures to be utilized to calculate extensive correlation between the candidate posture and the various baseline postures.

In another embodiment, the orthogonal relationship between the X, Y, and Z axes may be used to decrease the number of calibration postures needed to quantify different postures. For example, the cross product vector A and vector B is defined as a vector C that is perpendicular (orthogonal) to both vectors A and B, with a direction given by the right-hand rule and a magnitude equal to the area of the parallelogram that the vectors span. The vector product of two vectors is a vector perpendicular to both of vectors A and B. The vector product magnitude is obtained by multiplying the magnitudes of the vectors by the sine of the angle between the vectors. The direction of the vector product can be determined by the corkscrew right-hand rule. In this manner, the orthogonal orientation may be used to estimate posture orthogonal to at least two other orthogonal postures using vector cross product. In one example, standing (A) and supine (B) posture vectors may be measured. The LLR posture vector may then be derived using the cross product:

$$ C'' = (A \times B) = (A_Y*B_Z - A_Z*B_Y, A_Z*B_X - A_X*B_Z, A_X*B_Y - A_Y*B_X) $$

During other activity (N), the vector inner product of C" and N vectors may then represent the extent of correlation between the candidate posture and the baseline LLR posture (1 to 1). In one example, the calibration posture may be used to estimate at least two other independent orthogonal postures by using a matrix rotation at 90-degree angle operations.

$$ B''' = \begin{bmatrix} 1 & 0 & 0 \\ 0 & 0 & -1 \\ 0 & 1 & 0 \end{bmatrix} \begin{bmatrix} A_X \\ A_Y \\ A_Z \end{bmatrix} = A_X - A_Z + A_Z \text{ and } C''' = \begin{bmatrix} 0 & -1 & 0 \\ 1 & 0 & 0 \\ 0 & 0 & 1 \end{bmatrix} \begin{bmatrix} A_X \\ A_Y \\ A_Z \end{bmatrix} = A_X - A_Z + A_Z $$

At 408, the one or more processors apply one or more thresholds to the CB posture relation values to identify a category of an actual or resultant patient posture from the candidate patient posture. Figure 4B illustrates a graphical example of a manner in which thresholds may be applied to the CB posture relation values. Figure 4B illustrates continuous values quantifying projection of a correlation between a candidate posture and first and second baseline postures (arranged along the horizontal and vertical axes). The vertical axis represents a degree/extent of correlation between candidate posture and a baseline supine posture, while the horizontal axis represents a degree/extent of correlation between candidate posture and a baseline RLR posture. For example, when a supine extent value 420 (e.g., the vector inner product of the candidate N vector and the baseline supine B vector) is between .95 and 1.1, the vertical posture is supine. Alternatively, when the supine extent value is between .6 and .95, vertical posture may be various degrees of inclined sitting 422. In another example, when the supine extent value is between .35 and .6, vertical posture may be upright sitting. When the supine extent value is between -.1 and .35, vertical posture may be upright standing 424. When supine extent value is between -1.1 and -.1, vertical posture may be various degrees of prone, or incline 425. With respect to the horizontal axis, when the RLR extent value 426 (e.g., the vector inner product of the candidate N vector and the baseline RLR D vector) is between .8 and 1.1 lateral posture may be right. Otherwise, when extent value is between -1.1 and .5, lateral posture may be LLR 428.

Additionally or alternatively, a third and/or a fourth axis may be utilized to determine a degree/extent of correlation between the candidate posture and other baseline postures. Optionally, different baseline postures may be selected based on various criteria. For example, at certain times of day, it may be determined to utilize certain baseline postures. For example, in the middle of the night, it may be desirable to utilize the baseline supine posture, and one or both of the baseline RLR posture and/or baseline LLR posture, given that the patient is expected to be laying down. Alternatively, in the middle of the day, it may be desirable to utilize the baseline standing posture and baseline supine posture, with less interest in the baseline RLR posture and/or LLR posture.

Returning to Figure 4A, when the category for the actual or resultant patient posture is determined, flow moves to 410. At 410, the one or more processors define and store the actual patient posture. The actual patient posture may be stored in a memory of an IMD, PDE, remote storage unit, etc. By storing the actual patient posture, the calculated patient posture may be compared to the actual patient posture to adjust variables used while calculating the CB posture relation to improve the calculations during future iterations of data collection.

In accordance with new and unique aspects herein, various techniques have been derived to conserve energy in connection with obtaining accelerometer signals. For example, embodiments herein need not simultaneously read accelerometer signals along all three axes, but instead may measure the accelerometer signals along different axes at different points in time.

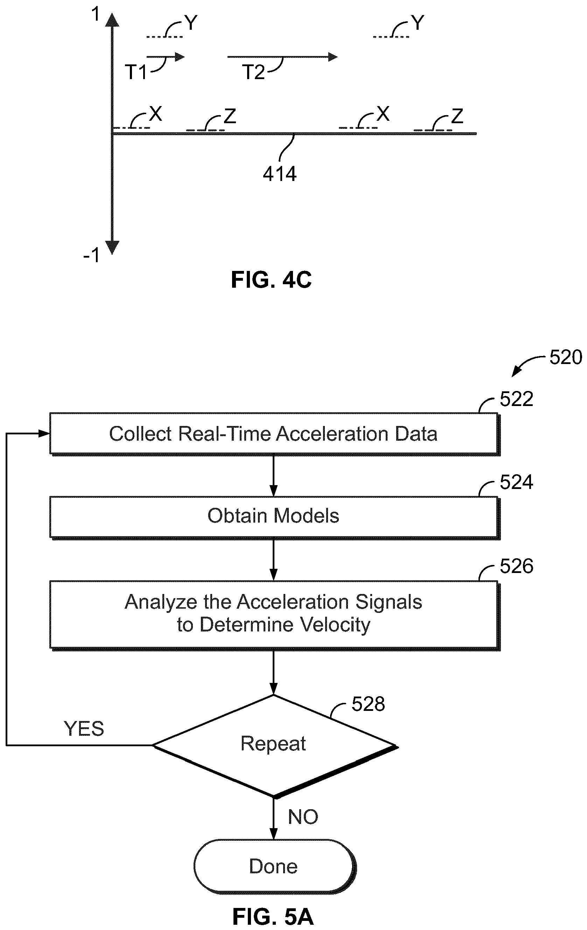

Figure 4C illustrates a graphical example of a timing pattern that may be utilized in connection with managing measurement of accelerometer signals in accordance with embodiments herein. The horizontal axis 414 represents time, while the markers X, Y and Z denote intervals of time during which accelerometer signals from the accelerometer are obtained for the corresponding axis. For example, accelerometer signals are initially obtained from the accelerometer output/channel that corresponds to the X axis of the accelerometer (also referred to as the X axis output or X axis channel). Thereafter, the device stops obtaining accelerometer signals from the X axis output/channel and switches to the output or channel corresponding to the y-axis of the accelerometer. During the time interval T1 accelerometer signals are obtained from the output/channel corresponding to the y-axis (also referred to as the y-axis output or y-axis channel). At expiration of the time interval T1, the device stops obtaining accelerometer signals from the y-axis output/channel and switches to the output or channel corresponding to the Z axis of the accelerometer. During a corresponding time interval, accelerometer signals are obtained from the output/channel corresponding to the Z axes (also referred to as the Z axis output or Z axis channel). At the expiration of the time interval for obtaining Z axes accelerometer signals, the device stops obtaining accelerometer signals from the Z axis output/channel.

The X axis is sampled for a first portion of a first data collection interval, while a second axis is sampled for a second portion of the first data collection interval that does not overlap with the first portion, and a third axis is sampled for a third portion of the first data collection interval that does not overlap with the first portion or second portion of the first data collection interval. A second data collection interval T2 may then be used to control the duty cycle of reading measurements.

In one embodiment, a same or common time interval may be utilized while obtaining X, Y and Z axes accelerometer signals. Additionally or alternatively, different time intervals may be utilized in connection with each of the X, Y and Z channels. For example, Y axis accelerometer signals may be obtained during a first time interval T1, whereas a different second time interval T2 may be utilized for obtaining X axis accelerometer signals and/or y-axis accelerometer signals. It is recognized that the order in which accelerometer signals are obtained from the various channels may vary. Also, one channel may be sampled more often than the other channels. For example, the X axis channel may be sampled twice as often as the Z axis channel, such as when the activity of interest or purpose for obtaining the accelerometer signals indicates that one axis may have more relevant information.

The X, Y and Z axis accelerometer signals are stored as an X axis accelerometer data set, a y-axis accelerometer data set and Z axes accelerometer data set. Thereafter, the accelerometer may enter an idle state for a desired time interval, such as the time interval T2. When the time interval T2 expires, the accelerometer may repeat the collection of accelerometer signals along the various channels. The time intervals may be managed in various manners. For example, a hardware timing circuit may be utilized to define the various time intervals T1 and T2. Additionally or alternatively, firmware may be utilized to set and countdown corresponding time intervals T1 and T2.

In accordance with new and unique aspects herein, it has been found that accelerometer signals need not be simultaneously obtained along all three channels. By managing the manner in which accelerometer signals are obtained in a serial manner, embodiments herein reduce the energy consumption utilized when monitoring posture. Also, in accordance with new and unique aspects herein, it is been recognized that it may take several seconds for a body posture to change in any notable degree. Also, steady-state postures are of more interest as compared to posture transitions. The foregoing recognition in the manner in which posture changes and the nature of information of interest regarding posture, it is been found that sampling posture measurements from each axis sequentially produces reliable information.

Various physical embodiments may be implemented to manage and accomplish collection of accelerometer signals along the various axes in a time (sequential) varying manner. The data collection interval may vary, depending upon the use of the information, the degree of specificity in posture of interest and other factors. The data collection may be initiated by a patient, clinician, automatically based on a schedule, determined condition, or threshold being met, or the like. The data collection interval may occur for a select period of time, until receiving a data collection halt instruction from a patient, clinician, automatically based on a schedule, determined condition, or threshold being met, etc., or the like. A halt instruction may be any signal received by an accelerometer, IMD, PDE, or the like that results in the accelerometer no longer obtaining data, and the data collection interval ending.

In yet another embodiment, energy may be conserved by managing the measurement duty cycle and/or by switching circuitry between awake and asleep states based on various criteria. For example, a processor or control circuitry for the accelerometer may reduce energy consumption by powering off some or all circuitry associated with obtaining and processing accelerometer signals between data collection intervals, also referred to as a posture acquisition duty cycle. The posture acquisition duty cycle may be increased by increasing the amount of time between accelerometer signal measurements. Alternatively or alternatively, the processor and/or control circuitry may power down (or set to an in active state) 2 two out of 3 accelerometer output channels, such that accelerometer signals are only obtained from a single channel periodically. The accelerometer signals from the single active channel may be sampled periodically and analyzed. The analysis may include determining whether the accelerometer signals from the active channel satisfy one or more criteria. For example, the condition may be whether the accelerometer signals for the single active channel exhibit a level of activity that is greater than a determined threshold. When the activity over the single active channel exceeds the threshold, the processor or control circuitry may wake up, activate, or simply begin to obtain accelerometer signals from one or both of the other two channels. Thereafter, two or more of the three channels may then be sampled simultaneously or serially (e.g. as described in relation to Figure 4) for a predetermined number to next duty cycles or for a period of time defined by other criteria (e.g. until an activity of interest has stopped. To this end, channels may be powered off during segments of low activity to further reduce energy consumption. For example, the device may be configured to operate at reduced energy and/or while monitoring only a single channel during an activity acquisition sleep time. During a determined period, when no measurement is desired, or when activity is unlikely, the processor and/or acquisition control circuitry may turn off for a determined interval of time. Alternatively, acquisition of accelerometer signals over 2 out of the 3 axes may be suspended and a single axis may only be sampled periodically (e.g. every ten minutes during a two hour interval) and when change is detected in the single axis measurement greater than a specific threshold, all activity channels are then sampled. Alternatively, activity channels may be suspended into low-power mode during segments of stable posture and switched to full power when a change of posture is detected.

The foregoing examples described somewhat independent analysis of the accelerometer signals captured over the various channels. Additionally or alternatively, acceleration signals from two or more of the channels may be combined, to form a composite activity signal, in certain instances to obtain certain types of information. For example, the composite activity signal may be formed by calculating an average value of the accelerometer signals from the X-axis, Y-axis, and Z-axis channels measured for a predetermined period of time (e.g. one second), where the three accelerometer signals are measured simultaneously.

The composite activity signal is independent of the orientation of the accelerometer. For example, the accelerometer may be positioned such that the Y-axis of the accelerometer predominantly measures the activity-related acceleration of the patient, whereas the X axis and Z axis accelerometer signals may provide very little information indicative of activity. Nonetheless, when all three signals are combined, the resulting composite activity signal affords a good indicator of overall patient activity regardless of the accelerometer orientation. In accordance with embodiments herein, methods and systems may utilize the composite activity signal to monitor activity intensity or activity tolerance, including the identification of positive or negative trends therein.

Optionally, methods and systems herein may identify one or a subset of the accelerometer channels that may be better suited to provide long term monitoring. For example, during implantation, or the days following implantation, a processor of an IMD or control circuitry of the accelerometer may cycle through the accelerometer signals from the X, Y, and Z axes channels. The accelerometer signals from the various channels are analyzed to identify the channel that exhibits a characteristic of interest, such as the largest amount of activity, the most frequent changes in activity, a signal level that is the more accurate indicator of particular types of patient activity and the like. The identify channel may then be designated as the primary or base channel that is utilized for long-term monitoring, while the other channels are set to an in active state or a sleep mode.

While the prior discussion is in connection with analyzing one patient based on baselines that are derived from that individual patient, optionally, in other embodiments, the real time accelerometer data sets may be utilized in combination with data sets from a population of patients to determine patient related data. In particular, by using the real-time acceleration activity data and population data sets of similarly patients, embodiments herein may track a velocity that a patient is traveling (e.g., walking, running) over a given time period in order to provide TR parameters.

Figure 5A illustrates a method 520 for calculating a TR parameter based on MD accelerometer data in accordance with embodiments herein. For example, the TR parameter may correspond to a velocity that a patient is traveling during a patient activity over a period of time. The determination of the TR parameter (e.g. velocity) may be made continuously over the course of a day, during a determined period such as during sleep, during an activity, or the like. The operations of Figure 5A may be implemented entirely within an implantable medical device, a local external device and/or remote server. Additionally or alternatively, the operations of Figure 5A may be distributed between the implantable medical device, a local external device and/or remote server.

At 522, one or more processors obtain real-time MD accelerometer data. In one embodiment, an accelerometer may be used to detect acceleration signals related to the activity or posture of a patient. The MD accelerometer data may include accelerometer signals may be obtained from more than one axis/channel. In another example, the acceleration signal may be received from orthogonal X, Y, and Z axes/channels. The acceleration signals may be obtained during a predetermined event or time period. For example, the acceleration signals may be obtained during a period of time when the patient is expected to be sleeping (e.g. between midnight and 2 AM). Alternatively, the acceleration signals may be received as a result of a determined event, such as a movement above a threshold activity level (e.g. while walking, during exercise, while jogging, while climbing stairs). In another embodiment, the acceleration signals may be received during an event such as a prescribed activity program, such as a six minute walk test.

At 524, one or more processors obtain one or more models correlating accelerometer data to the TR parameter (e.g. velocity). For example, the one or more models may be developed from a patient population for which various real time accelerometer data is obtained and correlated to velocity. In one embodiment, the one or more models may be obtained from a remote database that may be located at a hospital, clinic, doctor's office, government agency, or the like and in communication with the one or more processor by a wire connection, wireless connection, cellular connection, cloud based connection, digital communication, analog communication, or the like. The models may be built by storing accelerometer data sets associated with a standing posture while a patient walks or jogs at known speeds. The known speed is entered in connection with a corresponding portion of the accelerometer data sets. Additionally or alternatively, the model may be implemented as an algorithm, mathematical equation, mathematical function, a look up table, a decision tree, or the like. The models may include, in addition to accelerometer data sets, patient physiological data, such as patient age, weight, height, BMI, tobacco use, alcohol use, previously diagnosed health conditions, previous surgeries, etc. For example, different models may be maintained for different patient demographics.

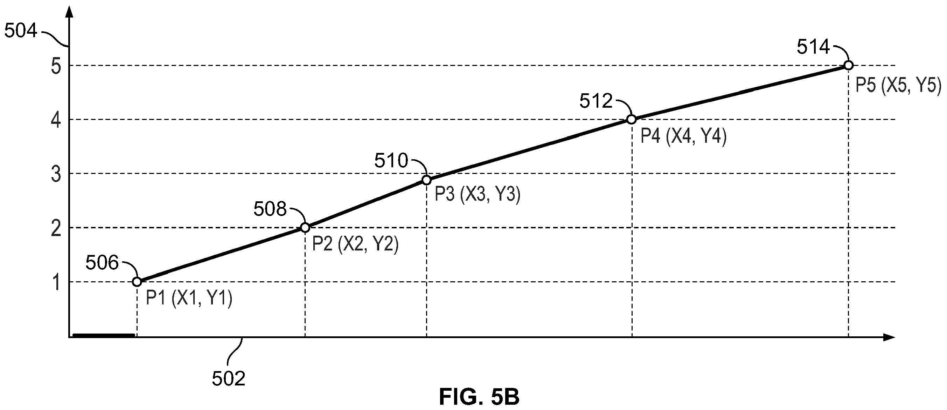

Figure 5B illustrates a model for calculating a travel -related (TR) parameter from instantaneous, or real-time, MD accelerometer data (e.g. accelerometer signals obtained along one or more axis). The model represents a count-velocity model that may be built for a patient or patient population. The count-velocity model correlates a "count" of activity to various velocity levels. The count may correspond to various characteristics of interest (COI) from the accelerometer signals. For example, the count may correspond to a number of steps taken by the patient, as the TR parameter. Additionally or alternatively, the count may correspond to a number of positive or negative peaks in the accelerometer signals, a number of zero crossings in the accelerometer signals, a number of turns in the direction of the accelerometer signals, an "area under the curve" in rectified accelerometer signals and the like. The count of steps or another TR parameter may be derived separately for each of the X, Y, and Z accelerometer signals or derived for a sum, a weighted sum or other mathematical combination of two or more of the accelerometer signals.

Values for the count (also referred to as step count) are correlated to particular levels for the TR parameter (e.g. velocity levels), such as by having patients walk at a given pace/velocity while measuring the accelerometer signals and determining the step count or other TR parameter of interest. In the example of Figure 5B, the control points 506 - 514 correlated step counts P1 - P5 to velocities 1-5mph, respectively. The control points can be patient specific based on patient demographics, including height, weight, gender, etc. of the patient. The control points 506-514 can be constant or they could be adjustable during in-clinic follow-up or auto-adjusted during long-term monitoring. In an alternative embodiment, the control points 506-514 may be determined only from monitored data related to the patient, and not based on population data. In another embodiment, the control points may be adjusted based on additional information received from a remote database, based on changes in the patient's physiological state, including age, weight, BMI, or like, based on changes in the patient's health status, including experienced heart attacks, strokes, surgeries, or the like, etc.

Returning to Figure 5A, at 526, the one or more processors analyze the acceleration signals to determine a step count or count of another accelerometer signal COI. In one embodiment, the analysis is applied to real-time acceleration signals, such as accelerometer signals obtained for a relatively short period of time (e.g., 30 seconds, 1 minute, etc.). In another embodiment, the step count may be obtained (e.g., for 30 seconds) and combined with a prior step count, such as through averaging or another mathematical combination.

The analysis at 526 may be applied to the individual accelerometer signals obtained along the X, Y and Z axes/channels. For example, the X axis accelerometer signals may be analyzed with respect to one or more corresponding X axis models, the Y axis accelerometer signals may be analyzed with respect to one or more Y axis models, and the Z axes accelerometer signals may be analyzed with respect to one or more Z axis models.

Additionally or alternatively, the accelerometer signals from the various axes/channels may be combined to form a composite activity signal. For example, the X, Y and Z axes accelerometer signals may be summed with one another or otherwise combined through various mathematical functions to form the composite activity signal. As one option, different weights may be applied to the X, Y and Z axes accelerometer signals, such as when it is desirable to place more importance on signals obtained along one axis as compared to the others. The composite activity signal is then compared to one or more corresponding models to derive a corresponding velocity. The velocity information is saved in memory.

Optionally, the operations of Figure 5A may stop after the analysis at 526. Alternatively, in some instances, the process may not yet acquire a sufficient amount of accelerometer signals to accurately calculate a velocity at 526. Additionally or alternatively, the velocity calculator to 526 may represent an initial candidate velocity, but it may be desirable to obtain additional accelerometer signals to verify the velocity. Accordingly, flow may move to 528.

At 528, the one or more processors determine whether to repeat the process. Based on the determination, flow returns to 522 or the operations Figure 5A end. For example, the one or more processors may determine whether a prescribed activity program has ended. Additionally or alternatively, the one or more processors may determine whether sufficient accelerometer signals and/or counts have been obtained or whether additional accelerometer signals should be obtained. For example, a certain amount of information may be needed before a reliable velocity can be determined.

When more accelerometer signals and/or counts are needed, flow returns to 522 and more accelerometer signals are obtained. Alternatively, when sufficient accelerometer signals have been obtained, at 528, the one or more processors determine a velocity that corresponds to the accelerometer signals and count. For example, the velocity may be determined simply by referencing the model illustrated in Figure 5B.

Additionally or alternatively, the velocity may be derived from the accelerometer signals and/or step count without use of the model of Figure 5B. For example, as velocity increases the amplitude of an accumulated 1-sec activity signal increases. Hence, the accumulated 1-sec signal can be used as a surrogate for velocity. For example, if $X2<M1<X3$, velocity at $M1 = (Y3-Y2)/(X3-X2)*(M1-X2)+Y2$.

The process of Figure 5A may be implemented in connection with a prescribed patient activity, such as a six minute walk test. Additionally or alternatively, the process may be repeated continuously or periodically over an extended period of time to accumulate velocity information, such as for 1 or more complete days. The accumulated velocity measurements can be converted into distance travelled by summing valid accumulated velocity measurements over the period of time.

The number of steps travelled in 1 day can subsequently be calculated by dividing total distance travelled by an estimate of step length (e.g. for a patient population 2250 steps per mile of distance). Alternatively, the estimate of steps per mile can be adjusted for individual patient height (e.g. steps per mile = -13.4* (height in cm) + 4592).

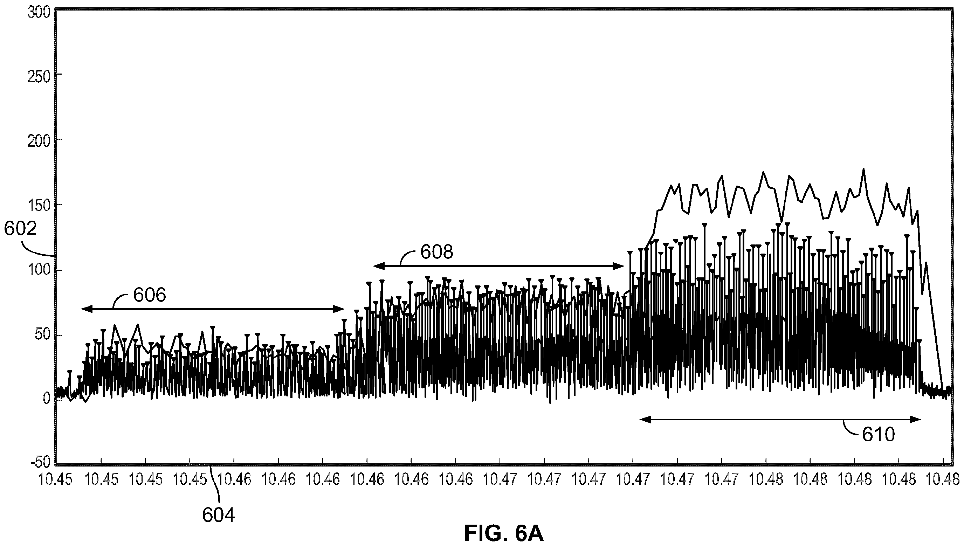

Figure 6A illustrates an alternative model for calculating a TR parameter from instantaneous, or real-time, MD accelerometer data (e.g. accelerometer signals obtained along one or more axis). The calculation of the TR parameter is based on the number and/or amplitude of signal peaks in a segment of accelerometer signals for a patient population, or individual patient data set. For example, the TR parameter may correspond to a number of steps taken during an interval of time and/or an average velocity over an interval of time. Figure 6A shows an example of instantaneous accelerometer signals 602 obtained along the Z axis during velocity data collection intervals 604. The data collection interval 604 includes three segments 606, 608 and 610, each of which illustrates a representative amount of activity within the Z axis accelerometer signal for different velocities. As a nonlimiting example, the segments 606, 608 and 610 may correspond to one minute intervals of time. For example, the activity level within the Z axes accelerometer signal during segments 606 may be determined to correspond to a velocity of one mile-per-hour and/or 75 steps per minute. The activity level within the Z axes accelerometer signal during segments 608 may be determined to correspond to a velocity of 2 mph and/or 90 steps per minute. The activity level within the Z axes accelerometer signal during segments 610 may be determined to correspond to a velocity of 3 mph and/or 110 steps per minute.

The accelerometer signals in Figure 6A correspond to accelerometer signals obtained along a single axis/channel from accelerometer. Additionally or alternatively, the accelerometer signals obtained along two or more of the X, Y and Z axes/channels may be separately analyzed for the number of peaks. Additionally or alternatively, the accelerometer signals along two or more of the axes/channels may be combined to form a composite activity signal, for which peaks are identified and utilized to calculate a number of steps.

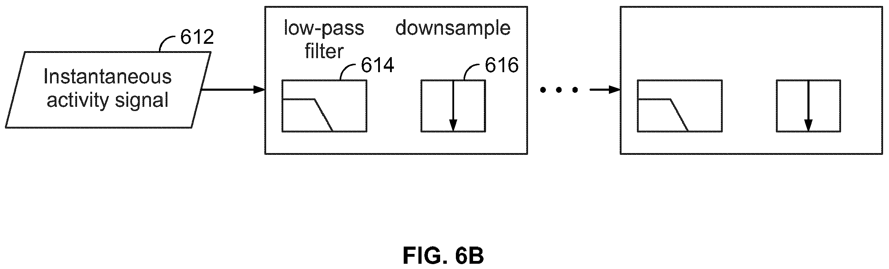

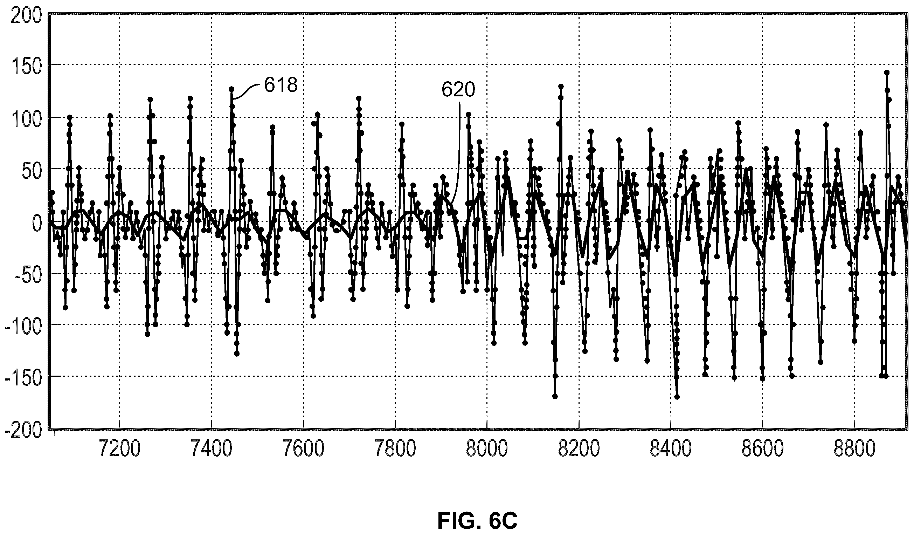

Figure 6B illustrates a schematic diagram for an accelerometer measuring system in accordance with embodiments herein. As illustrated in Figure 6B, and accelerometer 612 may output instantaneous accelerometer signals (also referred to as activity signals). The activity signals are supplied to a low-pass filter 614, an output of which is supplied to a downsampling circuit 616. In one example, decimation may be used to condition the accelerometer composite activity signal for subsequent processing to count a number of steps. By using a digital low-pass filter 614 that in one example includes a 5-10 Hz cut-off frequency (or multiple filter stages in series) coupled with down-sampling 616 (figure 6B), the low frequency component of steps (figure 6C) may be extracted.

Figure 6C illustrates a graph for an example activity signal measured along a Y axis during corresponding patient activity in accordance with embodiments herein. In Figure 6C, the accelerometer signal 618 is sampled at a rate of 100Hz. The accelerometer signal 618 is passed through the low pass filter and downsampling circuit of Figure 6B to form a decimated accelerometer signal as noted at 620. The decimated signal 620 has a frequency of approximately 5 Hz. By downsampling the accelerometer signal to a decimated accelerometer signals 620, embodiments herein reduce the power utilized by the processing circuitry. A zero-crossing algorithm or reversal points algorithm can be used to determine the number of steps from the 5 Hz accelerometer signal 620, without a need to further analyze the larger amount of data in the 100 Hz accelerometer signal 618, to achieve savings in power and cost. Additionally, the decimated signal could be performed continuously in hardware or on-demand in firmware.

Optionally, embodiments herein may utilize a step counting algorithm as described in relation to figures 6A-6C to calibrate the control points of the mapping function in the embodiment illustrated by the graph of Figure 5B. For example, the steps are counted for a specific time duration, and based thereon, the methods and systems estimate velocity during walking. For example, the velocity may be calculated based in the following equation:

$$\text{Velocity} = \frac{\text{distance travelled}}{\text{time duration}} = \frac{step\ length * number\ of\ steps}{\text{time duration}}$$

Step length can be estimated as a constant average human walking step length of a determined distance, that in one example may be 15 inches. Step length alternatively may be calculated from a population-wide formula, or population data set, considering height (step length in inches = 0.265*height in inches), age or health status. Using step length and time duration, Y values of control points 506-514 can be customized to each subject's own mapping. Additionally, the amplitude of steps varies proportionally with the velocity as shown in Figure 5B. In addition to the number of steps, the length of steps may be used to improve the control points as well. The advantage of this approach is to conserve energy of performing step counting continuously and only use on-demand as needed to calibrate the piecewise function that maps the 1-sec accumulated activity signal to estimated velocity.

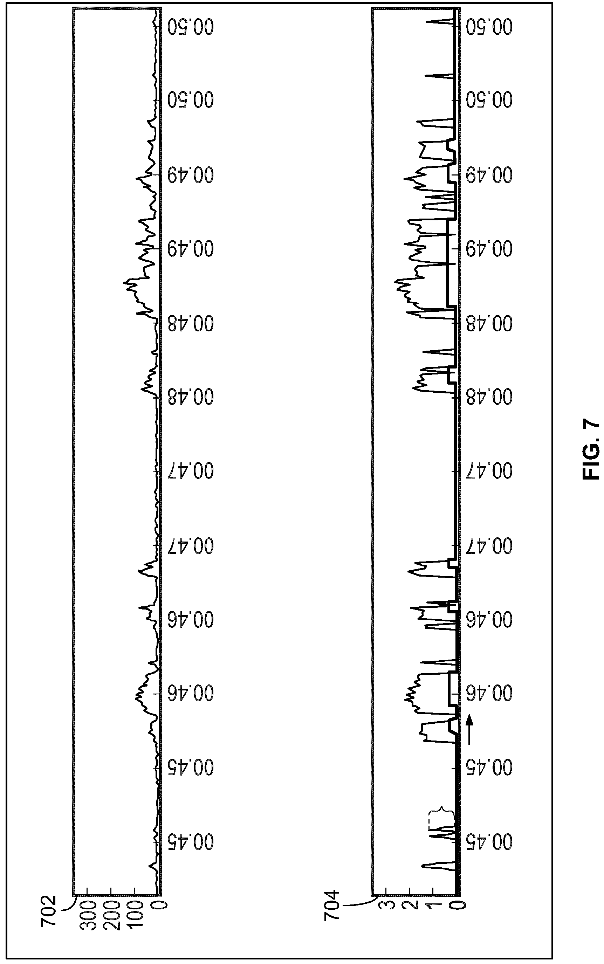

Figure 7 illustrates another example of a process for analyzing acceleration signals, such as in connection with the analysis at 526. Figure 7 illustrates a graph of accelerometer readings 702 and estimated velocity 704 over time 706. Sudden activity signal movement due to motion artifact or non-physiologic vibrations can be sensed by the accelerometer. Therefore, in the present embodiment, the sudden activity signals are filtered out. Specifically, activity measurements above a sustained activity amplitude threshold (SAAT) for a sustained activity time duration (SATD) are included in the distance calculation. In Figure 7, the top panel shows an example of 1-sec accumulated activity signal, while the bottom panel shows a valid movement filter (green) based on SAAT = 15 counts and SATD = 5 secs. The total estimated distance travelled is estimated as the integral of the velocity time plot (sum of estimated velocity * Δtime). Activity measurement could be any individual accelerometer activity axis or a linear combination of accelerometer axes signals (e.g. average of X, Y, Z composite activity signal). In this manner, activity intensity or activity tolerance may be based on the composite activity signal. To this end, the analysis of the obtained accelerometer data more accurately reflects the velocity of the patient over time.

With reference back to Figure 5A, at 528, a decision is made regarding whether velocity can be determined based on the analysis. If analysis results are inconclusive based on the accelerometer data obtained, the flow moves to the left and back to 524 to obtain addition real-time accelerometer data signals. If sufficient information is provided from the obtained accelerometer data, patient velocity for the period may be determined and the method is done.

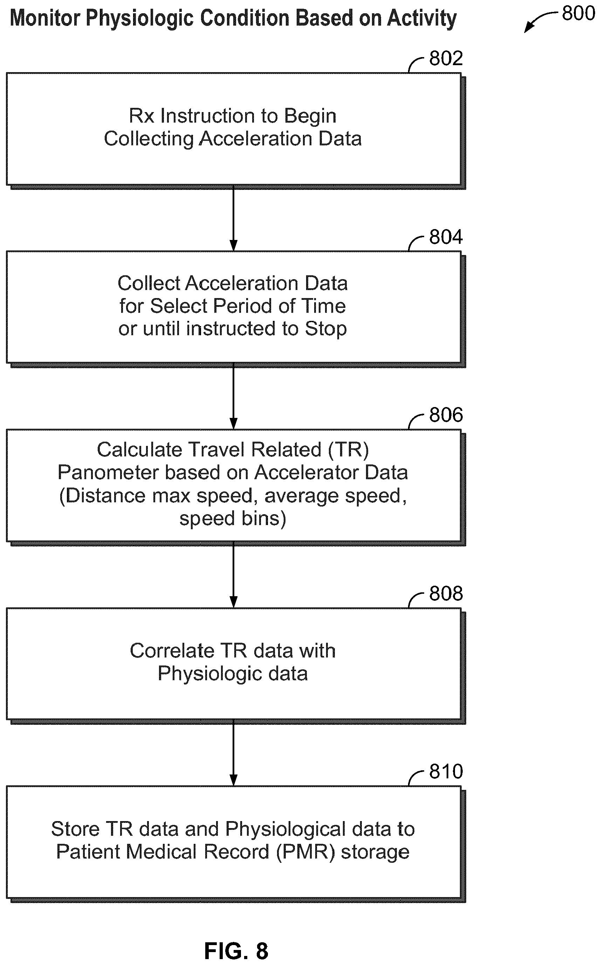

Embodiments have been described in connection with analyzing one patient based on baselines that are derived from that individual patient, and determining patient velocity based on patient activity. These methods may be used to obtain TR parameters for use in the method of figure 2. Figure 8 is a specific example of how the method of figure 2 may be used by a physician administering a six minute walk test. Specifically, the accelerometer of an IMD of a patient may be used to monitor the patient at their home, away from a clinician's office. The risk weighted composite index may be determined by one or more processors of the IMD, an in home medical device, PDE, etc. and transmitted to a physician for treatment diagnosis.

Figure 8 illustrates a flow block diagram of a method 800 of monitoring the physiologic condition of a patient based on activity using a monitoring system. In one example the monitoring system is the monitoring system of Figure 1.

At 802, one or more processors provide an instruction to begin obtaining accelerometer data, including MD accelerometer data. In one example, a patient may be conducting a test to monitor their heart. In one embodiment, the test is a six-minute walking test where a patient walks for six-minutes. Patient TR parameters such as speed, distance walked, reduction in walking pace over the six-minutes, heart rate, breathing, etc. may be monitored during the test and compared to previous iterations of the patient performing the six-minute walk. To this end, the patient may instruct a monitoring system that posture monitoring should occur when the six-minute walking test begins. In one example, the patient may have a PDE that is in communication with an IMD, and/or accelerometer such that when the patient activates an application in the PDE to monitor the accelerometer reading, the accelerometer, either through the IMD, are through the accelerometer itself, begins transmitting accelerometer data to the PDE. Specifically, changes in posture during the prescribed activity program, six-minute walking test, may be indicative of a health condition.

While in one embodiment an individual may manually start the monitoring of a test, in other embodiments, a timer may be provided such that a patient is constantly monitored during a determined data collection interval. For example, posture changes during sleep may be indicative of health conditions. So, every night between 2am and 4am, a timer in the PDE may automatically command the accelerometer to begin monitoring the posture of the patient for a period of two hours. Alternatively, an individual may have a routine where they exercise during the same hour every night. Again, an automatic time, or manual start either one may be used to begin obtaining accelerometer data for the determined data collection interval.

At 804, the accelerometer data is obtained for a select period of time or until instructed to stop with a halt instruction. After collection begins, the collection may continue for a select period of time. In one example, when prescribed activity program is being conducted, the patient may manually actuate an application on the PDE to both start and stop the collection of data. Alternatively, the application may have a timer that synchronizes with a tread mill, or similar walking device to automatically stop after the prescribed activity program. In another embodiment such as when monitoring is conducted when a patient is asleep, the accelerometer data may be obtained for a determined period, such as 2 hours, with the application of the PDE both beginning the two hour period of accelerometer data collection and ending the period.

At 806, TR parameters are derived based on the accelerometer data. The TR parameters may include distance traveled, maximum speed, minimum speed, average speed, and the like. For example, the system may utilize speed bin and increment a count for a corresponding speed bin based on average speed over data collection intervals (e.g., one minute) during a six-minute walk, posture variance, etc. The accelerometer data obtained may be used to determine the current physiologic state of the patient as provided by the TR parameters. The current physiologic state refers to a state of the patient at a particular point in time, namely when the data is obtained. A series of values for the physiologic state are determined at various points in time, such that the series of values provide an indication of an overall physiologic condition over a period of time. For example, the physiologic condition may exhibit trends or changes that are improving or deteriorating based on changes in the instantaneous physiologic state over time.

In one embodiment, one or more processors calculates at least one TR parameter. The parameter may be calculated by using a lookup table, decision tree, algorithm, formula, mathematical equation or determination, etc. The one or more processor may be coupled directly to the accelerometer, to an IMD containing the accelerometer, a remote device such as a PDE in communication with the accelerometer, or the like. In one embodiment, only one TR parameter may be calculated, while in other embodiments, multiple TR parameters may be determined.

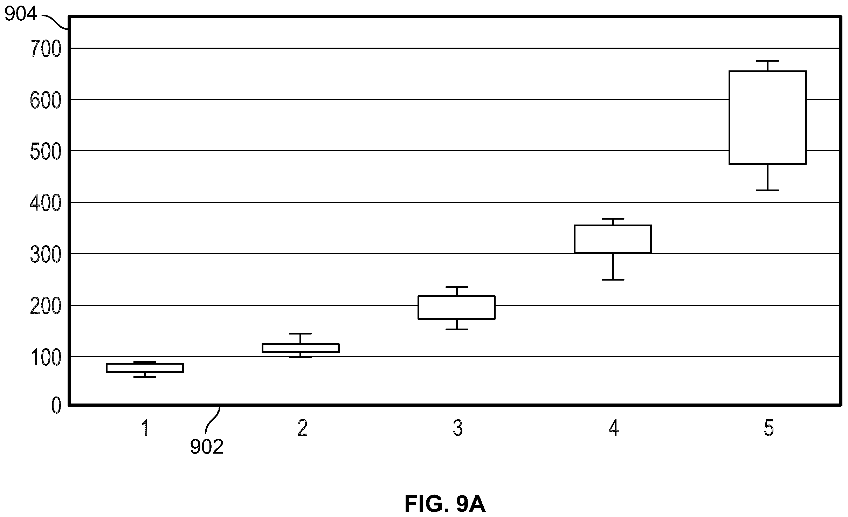

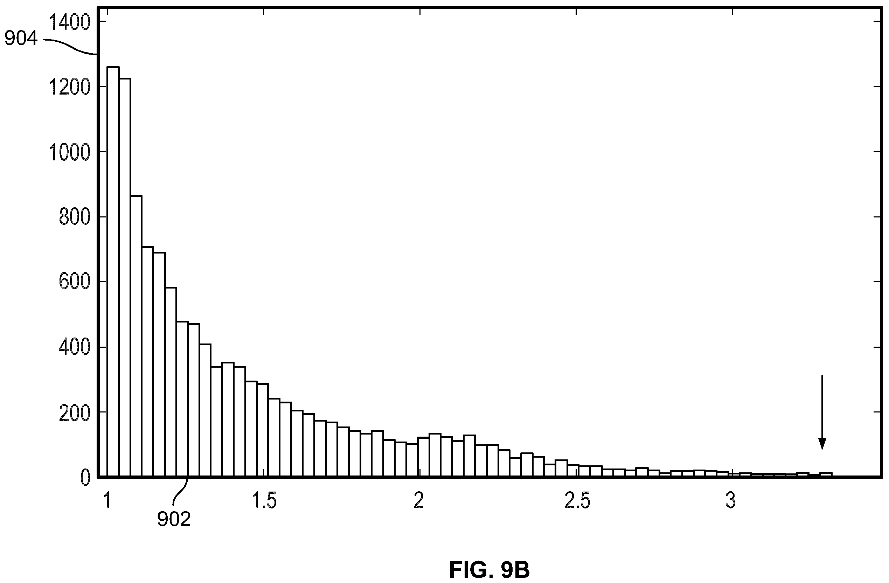

In one embodiment, the TR parameter determined is an activity profile of the patient. To this end, Figure 9A illustrates a graph of activity 902A monitored compared to a corresponding speed 904A of a patient. Meanwhile, Figure 9B illustrates a histogram of activity 902B corresponding to speed 904 over a 24-hour period.

The activity profile may be defined in various manners, such as through the graph (Fig. 9A) or histogram (Fig. 9B) that measure velocity 902 compared to activity signals 904. For example, an activity histogram may be divided into different speed ranges/windows such as every .1 mph ((e.g. 1mph to 1.1 mph, 1.1 mph to 1.2mph, 1.2mph to 1.3 mph). The profile/histogram maintains a count of an amount of time that the patient travels within the prescribed speed range. The count may represent an absolute count of time (e.g. 10 seconds, 30 seconds, one minute, five minutes). Additionally or alternatively, a count may be normalized to a defined interval of time (e.g. 30 seconds), such that each time the patient travels, for the pre-defined data collection interval, at a corresponding speed, the associated bin in the profile/histogram is incremented. For example, a count of 10 in the bin for 2mph to 2.1 mph would indicate that the patient walked at a speed between 2mph and 2.1 mph for 300 seconds (10x30) over the data collection interval during which the patient activity was tracked (e.g. over a six-minute walk or walking test, over the course of a day, a week, etc.).

With reference back to Figure 8, at 808, TR parameter is correlated with physiologic data. The current physiologic state of the patient may be correlated with physiologic data already provided by the patient. The physiologic data of the individual may be inputted into a processor, either at the accelerometer, the IMD, or at a remote PDE. Physiologic data may include patient age, height, weight, body mass index, previous health conditions, tobacco use, alcohol use, drug use, prescription drug use, family history, weight loss, previously recorded TR parameters, etc. By correlating the calculated TR parameter data with the physiologic data, treatment diagnosis of health conditions may be determined. Correlation may include use of a lookup table, decision trees, mathematical model or equation, computer modeling, algorithm, etc. In one example, the one or more processors may be configured to develop a motion model by obtaining and correlating multiple levels of baseline MD accelerometer data while a patient travels at corresponding baseline speeds.

For example, a patient may perform a six-minute walk test every day. The prescribed activity program for the six-minute walk test may have the patient perform the six-minute walk test at different speeds, such as one miles per hour (mph), 2mph and 3mph. In the example, on a first day, the six-minute walk test may occur at a first speed, on the second day, at a second speed, on the third day, a third speed, on the fourth day the first speed again, and so on. Each speed consequently has a corresponding baseline speed from which MD accelerometer data is obtained. The motion model may then correlate the different speed levels to one another to predict and/or diagnose the health condition of the patient.

In another example, a subset of parameters may be used as a basis with additional parameters to be added. Consequently, the subset always contributes to the algorithm. Still, other parameters may be used only as confirmatory roles.

Additionally, parameters available in an ICM may be different than the ones from CRT or ICDs. Depending on the type of device, a different set of parameters may be used. It is also may be possible that one method of combination is more preferable depending on the type of device.

At 810, TR parameter and physiologic data is stored a patient medical record (PMR) storage. The TR parameter and physiologic data may be stored in a memory coupled to the accelerometer, memory coupled to an IMD, a memory within a PDE, or the like. The recorded TR parameter may then be used for a comparison for future activities or test. In one embodiment, the TR parameter and physiologic data may also be transmitted and stored in a remote database for use by remote processors to use in diagnosing other patients. By monitoring, and storing the changes in activity of the patient, potentially adverse health conditions such as heart failure (HF), stroke, arrythmia, heart attack, heart disease, etc. may be diagnosed or predicted to allow preventive measures to be undertaken for the patient. To this end, monitoring and treatment diagnosis may be performed by a PDE, resulting in early detection and treatment of these conditions.

In one embodiment, a PDE, such as a smart phone, includes than application that communicates with an accelerometer in a cardiovascular implantable electronic device (CIED), such as an IMD. The smart phone may prompt the patient to initial walking. The accelerometer may then begin obtaining multi-dimensional accelerometer signals during a data collection interval of six-minutes. Alternatively, instead of a select period of time such as six-minutes, the data collection interval may last until receiving a data collection halt instruction.

During a six-minute walk data collection interval, the accelerometer data may calculate a computed speed and six-minute walk distance (6MWD) traveled. The accelerometer data may then be communicated to the application. In one example, the computed or calculated accelerometer data may be received. In another embodiment, the application may receive raw multi-dimensional accelerometer data, and the application calculates the average speed, and distance traveled based on a mathematical model. In one example, the mathematical model includes a determination of the acceleration determined by the accelerometer compared to the speed of a treadmill.

In another example, instead of directly measuring the 6MWD, a histogram of patient activity may provide an estimate of periodic (daily, weekly, biweekly, etc.) 6MWD. Again, based on a speed algorithm comparing acceleration determined by an accelerometer compared to speed of the patient, the daily speed histogram of a patient may be obtained. In particular, the patient may undergo a wide range of speed (e.g. 1-3.4 miles per hour) throughout a day. In this manner, the highest speed activity may be used as a basis for calculating a 6MWD, even without performing the six-minute walk. The highest speed activity may often be the highest exertion during a day, wherein the highest speed of an individual may be correlated to a give distance associated with the 6MWD. To ensure that speed is sustainable, the high speed may be maintained for a determined data collection interval, such as thirty seconds.

As a result of using the MD accelerometer signals, and communicating signals or data to a PDE, the six-minute walk test can be performed by a patient at their home. The results may then be transmitted to a clinician for diagnosis of changes in a health condition prior to an appointment. In one embodiment, a risk weighted composite index may be determined utilizing a diagnostic algorithm such as for heart failure (HF) to also transmit to a physician. In the algorithm X, Y, and Z parameters may be combined to be given equal weight to contribute to the algorithm. So, X, Y, and Z may be given 33% weight within the algorithm. Alternatively, a subset of the parameters may be given more weight if the parameters correlate with a HF event better than others. So, if data illustrates, X may be given a weight of 50% towards the algorithm while Y and X are both given only 25%. For example, in HF with preserved ejection fraction (HFpEF), any parameters related to left ventricle (LV) function may not be important. As a result, activity or posture measurement may be a better indicator for worsening HF.

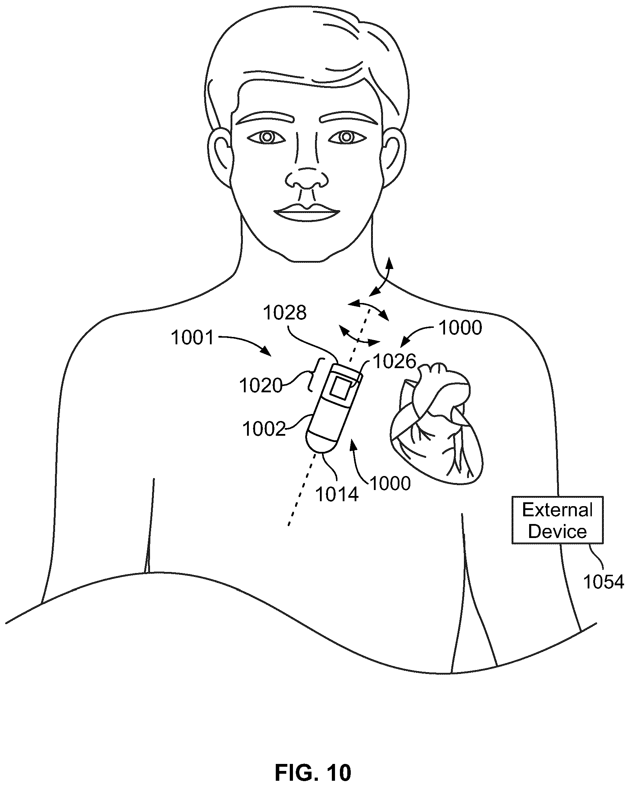

Figure 10 illustrates an example IMD 1000 intended for subcutaneous implantation at a site near the heart that may house a monitoring system 1001. In one example the monitoring system 1001 is the monitoring system described in Figure 1. The IMD 1000 is illustrated as exemplary only, and the monitoring system 1001 may be included in other systems. The IMD 1000 includes two or more spaced-apart sense electrodes 1014, 1026 positioned with respect to a housing 1002. The sense electrodes 1014, 1026 provide for detection of far field electrogram signals. The header 1020 includes an antenna 1028 and the electrode 1026. The antenna 1028 is configured to wirelessly communicate with an external device 1054 in accordance with one or more predetermined wireless protocols (e.g., Bluetooth, Bluetooth low energy, Wi-Fi, etc.).

The housing 1002 includes various other components such as: sense electronics for receiving signals from the electrodes, a microprocessor for analyzing the far field CA signals, including assessing the presence of R-waves in cardiac beats occurring while the IMD is in different IMD locations relative to gravitational force, a loop memory for temporary storage of CA data, a device memory for long-term storage of CA data, sensors for detecting patient activity, including an accelerometer for detecting acceleration signatures indicative of heart sound, and a battery for powering components.

The IMD 1000 may sense far field, subcutaneous CA signals, processes the CA signals to detect arrhythmias and if an arrhythmia is detected, automatically records the CA signals in memory for subsequent transmission to an external device 1054.

The IMD 1000 is implanted in a position and orientation such that, when the patient stands, the IMD 1000 is located at a reference position and orientation with respect to a global coordinate system that is defined relative to a gravitational direction. For example, the gravitational direction may be along the Z-axis while the X-axis is between the left and right arms.

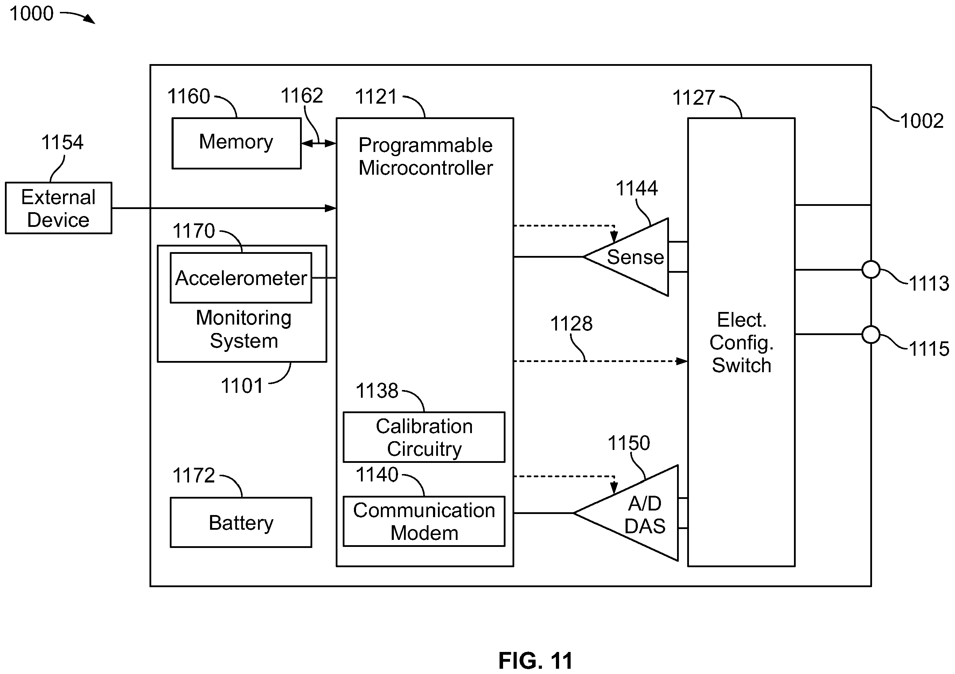

Figure 11 shows a block diagram of the IMD 1000 formed in accordance with embodiments herein. The IMD 1000 has a housing 1002 to hold the electronic/computing components. The housing 1002 (which is often referred to as the "can," "case," "encasing," or "case electrode") may be programmably selected to act as an electrode for certain sensing modes. Housing 1002 further includes a connector (not shown) with at least one terminal 1113 and optionally additional terminals 1115. The terminals 1113, 1115 may be coupled to sensing electrodes that are provided upon or immediately adjacent the housing 1002. Optionally, more than two terminals 1113, 1115 may be provided in order to support more than two sensing electrodes, such as for a bipolar sensing scheme that uses the housing 1002 as a reference electrode. Additionally or alternatively, the terminals 1113, 1115 may be connected to one or more leads having one or more electrodes provided thereon, where the electrodes are located in various locations about the heart.

The type and location of each electrode may vary.

A switch 1127 is optionally provided to allow selection of different electrode configurations under the control of the microcontroller 1121. The electrode configuration switch 1127 may include multiple switches for connecting the desired electrodes to the appropriate I/O circuits, thereby facilitating electrode programmability. The switch 1127 is controlled by a control signal 1128 from the microcontroller 1121. Optionally, the switch 1127 may be omitted and the I/O circuits directly connected via terminals 1113, 1115.

The IMD 1000 includes sensing circuit 1144 selectively coupled to one or more electrodes that perform sensing operations, through the switch 1127 to detect cardiac activity data indicative of cardiac activity. Optionally, the sensing circuit 1144 may be removed entirely, and the microcontroller 1121 perform the operations described herein based upon the CA signals from the A/D data acquisition system 1150 directly coupled to the electrodes. The output of the sensing circuit 1144 is connected to the microcontroller 1121 which, in turn, determines when to store the cardiac activity data of CA signals (digitized by the A/D data acquisition system 1150) in the memory 1160.

The IMD 1000 includes a programmable microcontroller 1121 that controls various operations of the IMD 1000, including cardiac monitoring. Microcontroller 1121 includes a microprocessor (or equivalent control circuitry), RAM and/or ROM memory, logic and timing circuitry, state machine circuitry, and I/O circuitry. The microcontroller 1121 is configured to implement the operations described herein in connection with obtaining and analyzing accelerometer signals.

The microcontroller 1121 may also include calibration circuitry 1138 that is configured to implement the calibration operations described herein. Among other things, the calibration circuitry 1138 obtains baseline accelerometer signals from an accelerometer 1170 in connection with specific patient postures. The postures may include supine, standing, laying on a right side, laying on are left side, angled, or the like. The calibration circuitry 1138 may also calculate synthetic baseline accelerometer signals based on orthogonal baseline accelerometer signals that are directly measured by the accelerometer 1170 as described herein. Although not shown, the microcontroller 1121 may further include other dedicated circuitry and/or firmware/software components that assist in monitoring various conditions of the patient's heart and managing pacing therapies.

The IMD 1000 is further equipped with a communication modem (modulator/demodulator) 1140 to enable wireless communication. In one implementation, the communication modem 1140 uses high frequency modulation, for example using RF, Bluetooth, or Bluetooth Low Energy telemetry protocols. The signals are transmitted in a high frequency range and will travel through the body tissue in fluids without stimulating the heart or being felt by the patient. The communication modem 1140 may be implemented in hardware as part of the microcontroller 1121, or as software/firmware instructions programmed into and executed by the microcontroller 1121. Alternatively, the modem 1140 may reside separately from the microcontroller as a standalone component, or external device 1154. The modem 1140 facilitates data retrieval from a remote monitoring network. The modem 1140 enables timely and accurate data transfer directly from the patient to an electronic device utilized by a physician.

By way of example, the external device 1154 may represent a bedside monitor installed in a patient's home and utilized to communicate with the IMD 1000 while the patient is at home, in bed or asleep. The external device 1154 may be a programmer used in the clinic to interrogate the IMD 1000, retrieve data and program detection criteria and other features. The external device 1154 may be a PDE (e.g., smartphone, tablet device, laptop computer, smartwatch, and the like) that can be coupled over a network (e.g., the Internet) to a remote monitoring service, medical network and the like. The external device 1154 facilitates access by physicians to patient data as well as permitting the physician to review real-time accelerometer data sets as obtained by the IMD 1000.

The microcontroller 1121 is coupled to a memory 1160 by a suitable data/address bus 1162. The memory 1160 stores the accelerometer signals, accelerometer data sets, reference posture data sets, cardiac activity signals, as well as the markers and other data content associated with detection and determination of the condition of the heart of the patient.

The IMD 1000 can further include one or more accelerometer circuits 1170. For example, the accelerometer circuits 1170 may be part of a monitoring system 1101, or may represent one or more accelerometers, such as a three-dimensional (3D) accelerometer. The accelerometer circuits 1170 may utilize a piezoelectric, a piezoresistive, and/or capacitive components are commonly used to convert the mechanical motion of the 3D accelerometer into an electrical signal received by the microcontroller 1121. By way of example, the 3-D accelerometer may three outputs/channels that generate three corresponding electrical signals indicative of motion in three corresponding directions, namely X, Y and Z directions. The electrical signals associated with each of the three directional components may be divided into different frequency components to obtain different types of information therefrom.

The accelerometer circuits 1170 obtain device location information with respect to gravitational force while the IMD obtains cardiac activity signals in connection with multiple cardiac beats. In one example, the accelerometer circuits 1170 include the accelerometer as described in relation to Figure 1. The microcontroller 1121 may utilize the signals from the accelerometer circuits 1170. While shown as being included within the housing 1002, the accelerometer circuit 1170 may be external to the housing 1002, yet still, be implanted within or carried by the patient.

A battery 1172 provide operating power to all of the components in the IMD 1000. The battery 1172 is capable of operating at low current drains for long periods of time. The battery 1172 also desirably has a predictable discharge characteristic so that elective replacement time can be detected.

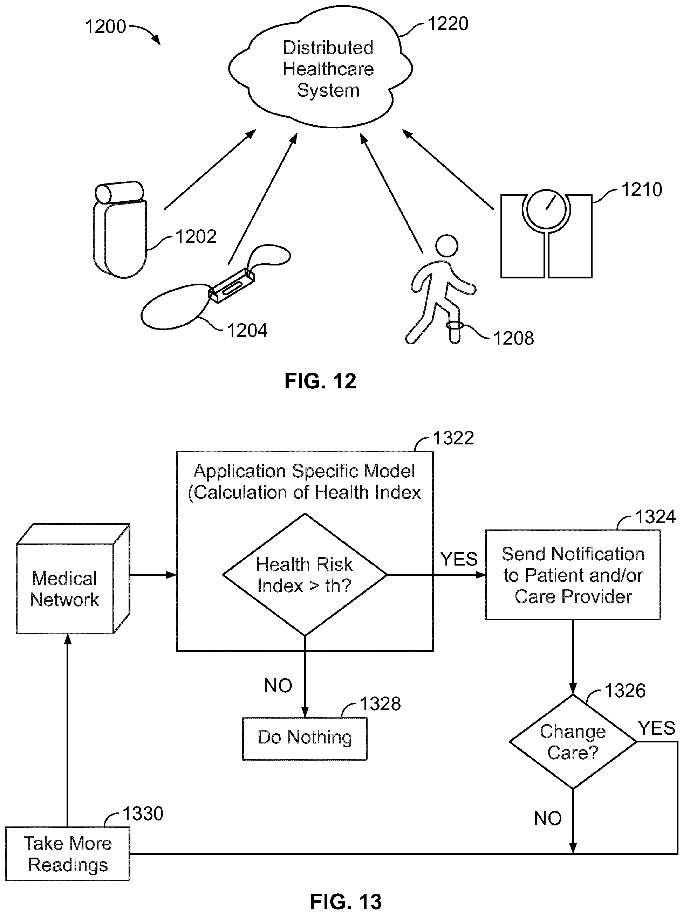

Figures 12 illustrate a digital healthcare system implemented in accordance with embodiments herein. The system integrates accelerometer signals and the other information derived from accelerometer signals with other health data in connection with monitor a patient condition, progression of a health condition, trends in a patient's health condition, treatment, changes in therapy/medication and the like. The healthcare systems may include wearable PDE that communicate with an IMD or accelerometer and a remote database. As a result, the healthcare system may monitor health parameters of patient, including MD accelerometer data and TR parameters, and provide a treatment diagnosis for the patient based on the monitored health parameters.

The system may be implemented with various architectures, that are collectively referred to as a healthcare system 1220. By way of example, the healthcare system 1220 may be implemented as described herein. The healthcare system 1220 is configured to receive data from a variety of external and implantable sources including, but not limited to, active IMDs 1202 capable of delivering therapy to a patient, passive IMDs or sensors 1204, wearable sensors 1208, and point-of-care (POC) devices 1210 (e.g., at home or at a medical facility). Any of the IMD 1202, sensor 1204, and/or sensor 1208 may implement an accelerometer circuity and perform the analysis of accelerometer signals as described herein. The data from one or more of the external and/or implantable sources is obtained and transmitted to one or more secure databases within the healthcare system 1220. Optionally, the patient and/or other users may utilize a PDE device, such as a smart phone, tablet device, etc., to enter data. For example, a patient may use a smart phone to provide feedback concerning activities performed by the patient, a patient diet, nutritional supplements and/or medications taken by the patient, how a patient is feeling (e.g., tired, dizzy, weak, good), etc.

Figure 13 illustrates a high-level flowchart of a method, implemented by the medical network of Figure 12, for processing current or real time accelerometer data and/or IMD data in accordance with embodiments herein. The healthcare system includes one or more computing devices (e.g., servers, local external devices, MP devices) that are configured to obtain and process BGA data, accelerometer data and/or IMD data. Upon receipt of new (or changes in) BGA data, accelerometer data and/or IMD data, at 1322, the processor of the computing device(s) identifies one or more application specific models or ASM to analyze the BGA data, accelerometer and/or IMD data. Optionally, the analysis by the ASM may incorporate the additional BGA data, accelerometer and/or IMD data into any relevant trend tracked in connection with the present patient. The application specific model may be implemented in various manners, as described herein, including but not limited to lookup tables, decision trees, machine learning algorithms and the like. At 1322, the processor calculates a risk weighted composite index based on the incoming accelerometer and/or IMD data, alone or in combination with previously stored BGA data, accelerometer and IMD data. For example, a patient may perform a 6 minute walk test and the system may determine that the current 6 minute walk test took 20% longer than a prior walk test or an average for multiple prior walk tests. The increase in time to perform the walk test may represent a risk weighted composite index increase. At 1322, the processor also generates a treatment diagnosis based on the IMD data and accelerometer data. For example, the processor may adjust a patient's medication when the 6 minute walk test shows notable changes in the time it takes for the patient to complete the walk test.

At 1322, the processor also determines whether the risk weighted composite index exceeds one or more thresholds. When the risk weighted composite index does not exceed the threshold(s), the process interprets the condition as an indication that the incoming BGA, accelerometer and/or IMD data indicates that a patient's health condition remains relatively stable. This is assessed as either an instantaneous change relative to the last risk weighted composite index, or based on a gradual increase in the risk weighted composite index over a pre-defined period of time. Accordingly, flow moves to 1328 where the process determines that no other action is necessary. Alternatively, when the risk weighted composite index exceeds the threshold, the process interprets the condition as an indication that the incoming BGA, accelerometer and/or IMD data indicates that a patient's health condition is deteriorating and in connection there with flow moves to 1324.

At 1324, the processor generates a treatment notification based on the treatment diagnosis and directs the treatment notification to be sent to the patient and/or a care provider. At 1326, the one or more processors determine whether a change in care has been identified by the treatment diagnosis. Optionally, the operation at 1326 may be implemented manually by a clinician or other medical practitioner. As a further option, the operation at 1326 may be implemented automatically by one or more processors, as well as manually by a clinician or medical practitioner. The clinician or medical practitioner may then be afforded an option to "override" or modify the automated determination of a change in care.

At 1330, the processor determines whether to obtain additional BGA, accelerometer and/or IMD data and the process continues by obtaining additional data. For example, the operation at 1330 may simply represent a continuous loop at which the healthcare system 1220 waits to receive new/additional accelerometer and/or IMD data. Additionally or alternatively, the processor may determine (e.g., as part of the treatment diagnosis) that further data should be obtained before a change in care is decided. Optionally, the operation at 1330 may be implemented manually by a clinician or other medical practitioner. As a further option, the operation at 1330 may be implemented automatically by one or more processors, as well as manually by a clinician or medical practitioner. The clinician or medical practitioner may then be afforded an option to "override" or modify the automated determination to obtain additional accelerometer and/or IMD data. The process of Figure 13 automatically develops the treatment diagnosis (e.g., clinical insights) based on all available data. The clinical insights may result in a determination to i) obtain more data, ii) recommend a change in clinical care or otherwise. Optionally, a clinician may be afforded the option to "Opt-In" or "Opt-out" of one or more different features and applications, thereby allowing the clinicians to choose which clinical insights they receive in connection with managing patients. Optionally, a clinician may be afforded the option to make decisions (e.g., render a treatment diagnosis, change treatment, obtain more data) and/or validate/reject decisions rendered automatically by the one or more processors.

Optionally, the process of Figure 13 may be implemented in whole or in part within one or more accelerometer circuit, IMD, a PDE and/or a local external computing device. For example, an IMD may track the IMD data and detect possible deterioration of patient's health. When the IMD detects possible deterioration, the IMD may automatically obtain accelerometer data sets (when the accelerometer circuit is in the IMD). Additionally or alternatively, the IMD may instruct another IMD, a dedicated accelerometer circuit or other device to obtain accelerometer data sets. Additionally or alternatively, the IMD may instruct the patient to perform a prescribed physical activity and/or initiate another action in connection with obtaining supplemental accelerometer data. The combination of the IMD data, BGA data and the accelerometer data is analyzed in accordance with embodiments herein locally or remotely. Additionally or alternatively, the accelerometer may perform continuous monitoring for indications of possible deterioration in a patient condition. When the accelerometer identifies a possible deterioration in a patient condition, the accelerometer may automatically convey a device command to an IMD to direct the IMD to obtain supplemental IMD data. The combination of the accelerometer data and the supplemental IMD data is analyzed in accordance with embodiments herein locally or remotely.

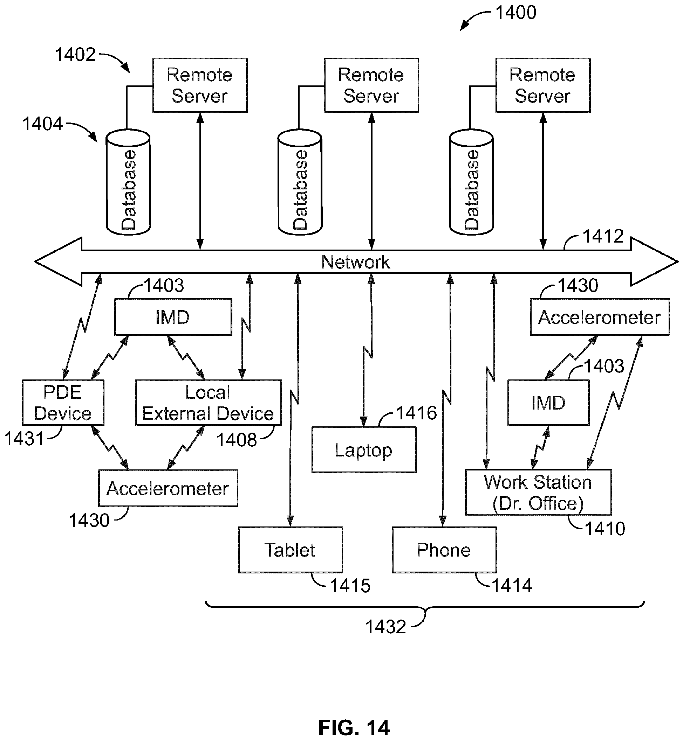

Figure 14 illustrates a healthcare system 1400 formed in accordance with embodiments herein. The healthcare system 1400 includes one or more servers 1402, each of which is connected to one or more database 1404. The servers 1402 and databases 1404 may be located at a common physical location and/or distributed between multiple remote locations within a city, state, country or worldwide. The servers 1402 communicate with one or more networks 1412. The network 1412 may be the internet, a voice over IP (VoIP) gateway, a local plain old telephone service (POTS) such as a public switched telephone network (PSTN), a cellular phone-based network, and the like. Alternatively, the network 1412 may be a local area network (LAN), a campus area network (CAN), a metropolitan area network (MAN), or a wide area network (WAM). The network 1412 serves to provide a network that facilitates the transfer/receipt of information such as IMD data, BGA data and accelerometer data.

The system 1400 also includes one or more IMDs 1403, one or more local external devices 1408, an accelerometer 1430, one or more PDE devices 1431 and one or more medical personnel (MP) devices 1432, all of which communicate (directly or indirectly) through the network 1412 to the servers 1402 and/or one another. The IMD 1403 may be passive or active, may obtain various types of data, such as cardiac electrical and/or mechanical activity data, PAP or other pressure related data, impedance data, RPM data, flow data, and the like. The PDE device 1431 may communicate with any or all of the IMDs 1403, local external devices 1408, an accelerometer 1430, as well as the network 1412. The PDE device 1431 obtains BRM data, such as based on manual inputs from a patient or other user, and/or based on automatic video and/or audio monitoring.

The local external device 1408 may be implemented as a variety of devices including, but not limited to, medical personnel programmer, a local RF transceiver and a user workstation, smart phone, tablet device, laptop computer, desktop computer and the like. The MP devices 1432 may also be implemented as a variety of devices including, but not limited to, medical personnel programmer, workstation, smart phone, tablet device, laptop computer, desktop computer and the like. Functionality of the MP device 1432 related to embodiments herein may be implemented through dedicated hardware circuits, firmware, software, and/or application operating on one or more computing devices.

The server 1402 is a computer system that provides services to other computing systems over a computer network. The servers 1402 control the communication of information including IMD data, patient entered data, medical record information and accelerometer data. The servers 1402 interface with the network 1412 to transfer information between the servers 1402, databases 1404, local external devices 1408, medical personnel devices 1432 for storage, retrieval, data collection, data analysis, treatment diagnosis, treatment recommendations and the like.

The databases 1404 store all or various portions of the information described herein, including, but not limited to, IMD data, accelerometer data, medical record information, treatment diagnoses and recommendations, and the like. Various portions of the information may be downloaded or uploaded in combination or separately to/from the databases 1404, local external devices 1408 and MP devices 1432. The local external device 1408 may reside in a patient's home, a hospital, or a physician's office. The local external device 1408 communicates wired or wirelessly with one or more IMD 1403 and/or accelerometer 1430. The servers and devices described herein may wirelessly communicate with one another utilizing various protocols, such as Bluetooth, GSM, infrared wireless LANs, HIPERLAN, 3G, satellite, as well as circuit and packet data protocols, and the like. Alternatively, a hard-wired connection may be used to connect the servers and devices. The local external device 1408, when implemented as a programmer, may be configured to acquire cardiac signals from the surface of a person (e.g., ECGs), and/or intra-cardiac electrogram (e.g., IEGM) signals from the IMD 1403. The local external device 1408 interfaces with the network 1412 to upload the data and other information to the server 1402.

Optionally, the local external device may represent a local RF transceiver that interfaces with the network 1412 to upload IMD data and/or accelerometer data.

The workstation 1410 may interface with the network 1412 via the internet or POTS to download various data, information, diagnoses, and treatment recommendations from the database 1404. Alternatively, the workstation 1410 may download raw data from the surface ECG units, leads, or monitoring device via either the programmer or the local RF transceiver. Once the user workstation 1410 has downloaded the cardiac signal waveforms, ventricular and atrial heart rates, or detection thresholds, the user workstation 1410 may process the information in accordance with one or more of the operations described above. The system may download information and notifications to the cell phone 1414, the tablet device 1415, the laptop 1416, or to the server 1402 to be stored on the database 1404.

Thus, provided is a distributed "digital" healthcare system that obtains various types of data, enables the data to be analyzed by various computing devices within the system and determines one or more treatment diagnosis and treatment recommendation substantially in real-time with the collection of new data. In this manner, unneeded and undesired hospitalizations may be avoided through preventative detection, reducing costs associated with emergency medical procedures. Additionally, such a system also assists in prolonging a human's life and increases patient care. Thus, an improved system and methodology are provided.

Optionally, the risk weighted composite index may be utilized to rank and schedule patients for future appointments to ensure those patients with the greatest risk of a medical emergency are monitored more closely than those with less of a risk.

The system may implement a method for obtaining new accelerometer data and based thereon, calculating a probability of a health condition in accordance with embodiments herein. The method may be initiated based on various criteria, such as periodically, continuously, at the direction of a clinician or network manager, a calendar reminder, patient activated, etc. Additionally or alternatively, the method may be initiated each time a test, such as a six-minute walking test is performed by a patient resulting in new accelerometer data. For example, each time an accelerometer detects activity from the six-minute walk test and sends the accelerometer data to a IMD, the IMD will send a new data upload request (with the patient ID and test results) to a server, database or other computing device of a medical network. As another example, the IMD, or accelerometer may wirelessly transmit accelerometer data to a local external device (e.g., a bedside monitor, laptop computer, smart phone, etc.) and the local external device then sends a new data upload request to the server, database or other computing device of the medical network. The upload request(s) may be utilized as one basis to initiate the method. As yet another example, an IMD, PDE device and/or accelerometer may initiate collection of additional IMD, BRM and/or accelerometer data.

Additionally or alternatively, the accelerometer may perform continuous acceleration monitoring and locally analyze the accelerometer data for indications of possible deterioration in a patient health. A processor determines whether new MD accelerometer data has been obtained. Optionally, on a weekly/monthly basis, patients may perform a point-of-care comprehensive blood panel test which is then transmitted to a central medical network. Optionally, the processor also obtains medication related information from the accelerometer data, where the medication related information may indicate changes in a patient's prescription regiment.

The processor determines a treatment diagnosis based on the accelerometer data. Optionally, the processor may classify the treatment diagnosis based on various predetermined criteria. The processor updates a patient medical record with the accelerometer data. Optionally, the processor may update the patient medical record to include historic information as appropriate based on patient history. Thereafter, the method may terminate without further action and/or, flow may move to one or more of the processes described herein for applying an application-specific model in connection with providing a treatment diagnosis and treatment recommendation.

The operations may be performed each time a patient performs activities, including during a six-minute walk test. Accordingly, the system described herein provides a patient with more flexibility, which should encourage better compliance to protocols. Further, by having a way to uniquely identify patient data is additionally advantageous. For example, data can be centralized, and analysis can be done with more assurance that all of the patient's data is being considered in the analyses. Embodiments herein may implement one or more of the methods and systems as described in U.S. Patent 9,760,679, titled "Data synchronization between two or more analyte detecting devices in a database".

For example, an index may represent a PAP systolic level, PAP diastolic level, PAP mean and the like. As another example, the IMD Index may represent a diuretic response profile that is calculated based on hemodynamic data (from the IMD data) and diuretic medication information. As another example, the IMD Index may represent PAP levels and/or trends derived from the IMD data. As another example, the IMD Index may represent ST segment levels and/or ST segment level shifts determined from cardiac activity data within the IMD data. As another example, the IMD Index may represent levels and/or trends in cardiac output, thoracic impedance, cardiogenic impedance, heart sounds and the like. As another example, the IMD Index may represent atrial fibrillation (AF) burden calculated based on CA signals indicative of AF episodes. The IMD Index may further represent MCS parameter levels and/or trends. Optionally, the processor may classify the IMD Index based on various predetermined criteria.

The processor updates the patient medical record with the IMD Index and any other information of interest from the new IMD data, including the raw IMD data. Thereafter, the method may terminate without further action and/or, flow may move to one or more of the processes described herein for applying an application-specific model to provide a treatment diagnosis and treatment recommendation.

Additionally or alternatively, the method may obtain new BRM data and based thereon, calculating a COI in accordance with embodiments herein. Additionally or alternatively, the method may be initiated each time a medical network receives a request from a PDE device or local external device to upload new BRM data. For example, the PDE device obtains BRM data continuously, periodically, in connection with select cardiac episodes. The PDE device will send a new data upload request (with the patient ID and BRM data) to a server, database, or other computing device of a medical network.

As yet another example, an IMD and/or accelerometer may initiate collection of additional IMD, BRM and/or accelerometer data. For example, an IMD and/or accelerometer may track IMD and/or accelerometer data and detect possible deterioration of patient health. For example, the IMD and/or accelerometer data trend may cross a threshold, change direction, or increase a downward trend. When the IMD and/or accelerometer detects possible deterioration, the IMD and/or accelerometer may notify the patient to enter BRM data through the PDE device. Optionally, when the IMD and/or accelerometer detects possible deterioration, the IMD and/or accelerometer may automatically convey a device command (as a treatment notification) to a PDE device. In response, the PDE device may automatically obtain BRM data.

The processor obtains the new BRM data and calculates a COI based on the new BRM data. Examples are discussed below for different physiologic COI that may be monitored in connection with different types of BRM data calculations. For example, the BRM data may track a sugar content of a patient's diet, with the characteristic of interest representing a sugar intake index. A patient's sugar intake may be analyzed in connection with blood glucose measurements and other accelerometer data, such as for diabetic patients. As another example, the BRM data may track a patient's exercise, with the characteristic of interest representing a calorie burning index. A patient's daily calorie expenditure may be analyzed in connection with trends in PAP levels (derived from IMD data) and/or activity levels indicative of heart failure behavior (derived from accelerometer data).

Optionally, the operations may be implemented when a patient does not actively enter BRM data. For example, a patient treatment/monitoring program may call for a patient to enter certain BRM data periodically (e.g., answering certain health, diet and/or exercise related questions daily or weekly). When a patient does not enter BRM data at the prescribed times or on a periodic basis, the method may record the non-entry of BRM data as "noncompliant BRM data." Additionally or alternatively, indirect BRM data may be derived from IMD data and/or accelerometer data. For example, select PAP trends may be indicative of certain patient behavior (e.g., weekend diet/exercise patterns). The fact that the patient is not transmitting direct BRM data at predefined periods indicates non-compliance. The fact that the PAP increases over the weekend may indicate high sodium/high calorie diet. Optionally, when an increase in the PAP trend is identified over a weekend, a potential in the increase of sodium/high calorie foods may be verified utilizing geo-location information for the patient (e.g., the patient went to a fast food restaurant), utilizing nutrition tracking application operating on a portable device and the like. When the select PAP trends are identified (with or without confirmation from geolocation information and nutrition tracking applications), the trend may be treated as indirect BRM data that is utilized to calculate a COI and stored in the patient medical record.

Optionally, the BRM data may be obtained from a third-party application and/or database. For example, numerous applications are implemented on smart phones and other user devices in connection with tracking diets and activity levels. Non-limiting examples of diet/nutrition tracking applications include "FATSECRET CALORIE COUNTER", "FOODUCATE", "LOSE IT!", "LOSE WEIGHT WITHOUT DIETING" applications, and the like. Non-limiting examples of activity tracking applications include the FITBIT application. In accordance with embodiments herein, the information obtained by third-party diet and activity tracking applications may be imported into a BRM data entry application operating on the same PDE or a different electronic device. Optionally, the information obtained by the third-party diet and activity tracking applications may be conveyed from the electronic device to a server implemented in connection with embodiments herein. As a further example, the third-party diet and activity tracking application may convey the diet and patient activity information directly to a remote server implemented in connection there with separate from the BRM data entry application operating on the PDE. The remote server may then convey the diet and patient activity information to the PDE device and/or server utilized in connection with embodiments herein to obtain, track, analyze and store BRM data.

As a further example, embodiments may utilize accelerometer data and/or IMD data as indirect indicators of BRM data. For example, the ASM may identify the fact that the patient is not transmitting BRM data at predefined periods, thereby indicates non-compliance. When the ASM does not receive direct BRM data, one or more other sources may be utilized as indirect BRM data. For example, when the IMD data includes PAP measurements, the ASM may be tracking the PAP trend (e.g., in connection with medical applications described herein). The ASM may utilize the PAP trend as indirect BRM data. For example, the ASM may determine that the PAP increases over a weekend and interpret the increasing PAP trend over certain times of week as an indicator that the patient has been following a high sodium/high calorie diet. Optionally, the determination that the patient is experiencing a high sodium/calorie diet can be verified using geo-location of the patient. For example, the ASM may obtain geo-location information from one or more electronic devices carried or otherwise controlled by the patient. A patient may utilize a wearable device (e.g., smart phone, tablet device, fit bit device, smart watch) that tracks a patient's location and periodically conveys geolocation information to a local patient monitoring device and/or remote server. The ASM may compare the patient's location to known locations of various types of establishments, such as fast food restaurants, ice cream shops and the like, and based thereon derive indirect BRM data. The ASM may use the geolocation information as a confirmation when an increase in the PAP trend is identified over a short period of time and during certain days of the week (e.g., a weekend). Additionally or alternatively, the ASM may obtain and analyze the geolocation information in parallel with or independent of PAP trend, and derive indirect to BRM data from the geolocation information independent of accelerometer data and/or IMD data. For example, a patient's diet and/or exercise history may be preprogrammed based on a series of questions answered by the patient. Optionally, the patient's diet and/or exercise history may be obtained over a period of time, such as through an application operating on an electronic device carried by the patient (e.g., a location tracking application). The ASM may include a geographic map with nodes for restaurants, gyms, and other establishments of interest. For example, the ASM may utilize the Google map database, or various other mapping applications and databases.

Optionally, the indirect BRM data may be derived from a food ordering application operating on a PDE device. For example, when a patient utilizes a smart phone to order carryout or delivery pizza, Chinese food, and the like, information regarding the food order may be conveyed to the ASM. The ASM may derive indirect BRM data from the online food order. In a simpler example, when the online food order includes a quantity of food associated with an individual consumer, the ASM may attribute all of the ordered food items to the patient. Alternatively, when the online food order includes a quantity of food associated with more than one consumer, the ASM may apportion a select quantity of the online food order to the patient's diet in various manners. For example, when the ASM is aware of a patient's "favorite foods", if the online food order includes the patient's favored foods, the ASM may identify the corresponding portion of the order and attribute the corresponding portion of the order to the patient's diet. Alternatively, when the online food order is for multiple people, but the patient's food preferences are not known/used, the ASM may assign a portion of the overall order to the patient (e.g., when an order is for four people, the ASM may assign one fourth of each food item to a patient's diet consumption record).

The foregoing examples regarding geolocation information and online food ordering applications are examples of how indirect BRM data may be obtained. Optionally, the patient may directly enter BRM data through the use of geolocation information and/or an online food ordering application. For example, when a patient goes to a fast food restaurant, the patient may designate the location (e.g., through dropping a geo-pin on a location tracking application, mark the restaurant through a diet tracking application, etc.). As another example, when a patient uses an online food ordering application, the patient may designate the food items that the patient plans to consume. The patient may designate the food items directly through the online food ordering application and/or through a separate diet/nutrition tracking application.

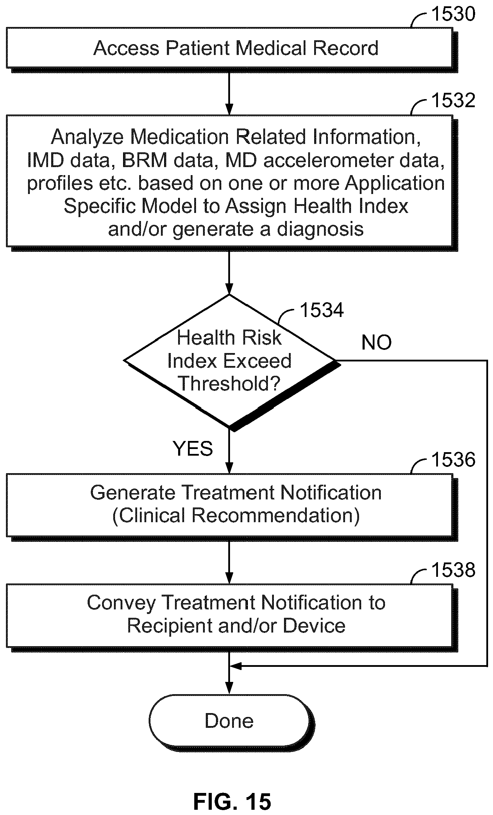

Figure 15 illustrates a method for generating a treatment diagnosis and treatment recommendation based on real time accelerometer data and IMD data in accordance with embodiments herein. The method of Figure 15 may be initiated based on various criteria, such as periodically, continuously, at the direction of a clinician, network manager or patient, in response to updates in a patient's accelerometer data and/or IMD data and the like.

At 1530, the processor accesses one or more patient medical records. The patient(s) medical record to be analyzed maybe identified in various manners. Additionally or alternatively, a physician or patient record manager may designate a patient medical record to be analyzed, such as in connection with an office visit, a telephone conversation, electronic mail, and the like. Additionally or alternatively, the process may be automatically initiated on a periodic basis for all patients and/or for patients who exhibit certain criteria. For example, the patient medical records, for patients known to have a certain health condition, may be analyzed daily, weekly, monthly and the like.

At 1532, the processor correlates a data entry time associated with collection of the medication related information, the IMD data, BRM data and accelerometer data. The processor analyzes the medication related information, the IMD data, BRM data and accelerometer data in connection with one or more application specific models (ASM) to generate a treatment diagnosis and calculate a risk weighted composite index related to the treatment diagnosis. The ASM may be implemented in various manners, including but not limited to threshold-based algorithms, template correlation algorithms, lookup tables, decision trees, machine learning algorithms and the like. Various embodiments are described for analyzing different combinations of IMD, BRM and accelerometer data in connection select medical applications. Various examples are also provided for treatment diagnosis that may be generated based on the analysis. The ASM analyzes data points from dissimilar data sources (e.g., from the IMD, BRM and accelerometer data) relative to one another to generate the treatment diagnosis. The data points from the IMD, BRM and accelerometer data have a relative level of importance with respect to one another, that varies, in connection with calculating the risk weighted composite index and generating the treatment diagnosis. The relative level of importance may vary in the context of a particular disease state or risk weighted composite index of interest.

In connection with generating a treatment diagnosis, the processor also assigns a risk weighted composite index that is indicative of a state or condition exhibited by the patient. Based on the type of medical application, the state of the patient may vary, as will the risks associated with the patient's state. As explained herein, the risk weighted composite index may be assigned a value along a scale, a value corresponding to a particular state or condition of the patient, and the like.

At 1534, the processor determines whether the risk weighted composite index exceeds a threshold. For example, when the risk weighted composite index is assigned values along a scale (e.g., 1-10), the threshold may be set at 6. Optionally, the determination at 1534 may be based on a relative change in the risk weighted composite index. For example, the risk weighted composite index would be determined to exceed the threshold when the risk weighted composite index is increased by 2 on the scale of 1-10, or when the risk weighted composite index is downgraded from moderate to severe. When the risk weighted composite index exceeds the threshold, the process interprets the condition as warranting a treatment notification, and accordingly, flow moves to 1536. When the risk weighted composite index does not exceed the threshold, the process interprets the condition to mean that the patient is maintaining a previous health state which does not warrant a new notification.

At 1536, the processor identifies a treatment diagnosis and treatment notification to be provided in connection with the risk weighted composite index. As one example, based on the MD accelerometer signals obtained during a six-minute walk test, a change in distance may be determined that increase the risk weighted composite index. As a result, medication dose increase may be recommended as a treatment diagnosis, and an electronic message, or displaying on a display accordingly may also be provided.

At 1538, the processor communicates the treatment notification to the corresponding one or more recipients and/or devices. For example, the treatment notification may represent an identification of a patient treatment diagnosis and treatment recommendation. The treatment diagnosis and treatment recommendation may be provided to the patient's physician or other medical practitioner. Additionally or alternatively, the treatment diagnosis and treatment recommendation may be provided directly to the patient. Examples of treatment notifications are discussed herein in connection with particular medical applications.

As explained herein, treatment diagnoses and recommendations may relate to changes in prescriptions, changes in parameters of an IMD and the like. Additionally, treatment diagnoses and recommendations may be implemented in connection with digital health/patient application features that are communicated to the patient through smart phone or another electronic device. The treatment notification may be provided to the patient and/or a caregiver, with the treatment notification representing educational material and/or feedback regarding negative health consequences of a patient's lifestyle choices when the combined data (BRM, IMD and accelerometer data) indicates trends in a negative direction. Similarly, the treatment notification may provide feedback regarding positive health consequences of a patient's lifestyle choices when the combined data indicates trends in a positive direction. As another example, when a patient with an IMD and/or accelerometer avoids exercise for a period of time, the notification may inform a patient that the patient's lack of exercise increases the likelihood of heart failure.

In accordance with one more aspects herein, a system for monitoring a physiologic condition of a patient may be provided, including an accelerometer configured to be implanted in the patient, the accelerometer configured to obtain multi-dimensional (MD) accelerometer data along at least two axes. The system may also include a memory configured to store program instructions, and one or more processors. When executing the program instructions, the one or more processors may be configured to initiate a data collection interval in connection with patient activity, obtain the MD accelerometer data during the patient activity for at least one of a select period of time or until receiving a data collection halt instruction, and calculate a travel-related (TR) parameter based on the MD accelerometer data. The one or more processors may also be configured to correlate the TR parameter with physiologic data obtained during the patient activity, and store the TR parameter and physiologic data as indicators of a current physiologic state of the patient.

Optionally, the system may also include an implantable medical device (IMD) that houses the accelerometer, first memory of the memory and at least a first processor of the one or more processors, where the first processor may be configured to obtain the MD accelerometer data.

Optionally, the first processor may be configured to implement at least one of the calculating of the TR parameter, the correlation of the TR parameter with the physiologic data, or the storing of TR parameter and physiologic data. In another aspect, the IMD may be further configured to wireless transmit, to an external device, at least one of the MD accelerometer data, the TR parameter, or the correlation of the TR parameter and the physiologic data. In another example, the system may further comprise an external device configured to wirelessly communicate with the IMD, the external device including a second memory of the memory and a second processor of the one or more processors, the second processor configured to implement at least one of the calculating of the TR parameter, the correlation of the TR parameter with the physiologic data, or the storing of TR parameter and physiologic data. In another aspect, the patient activity may represent a prescribed activity program.

Optionally, the TR parameter may represent at least one of a distance traveled or a speed, the one or more processors configured to calculate the TR parameter based on an activity profile for the corresponding patient.

Optionally, the one or more processors may be further configured to develop a motion model by obtaining and correlating multiple levels of baseline MD accelerometer data while a patient travels at corresponding baseline speeds. In another aspect, the calculating may include calculating a speed of movement based on the MD accelerometer data and the motion model. In another aspect, the motion model includes activity ranges associated predetermined speeds, the one or more processors further configured to bin the MD accelerometer data into the activity ranges and based thereon determine speeds traveled for sub-intervals of time corresponding to segments of the MD accelerometer data.

In one or more embodiments, a computer implemented method for monitoring a physiologic condition of a patient may be provided that may include utilizing one or more processors for, initiating a data collection interval in connection with patient activity, and obtaining multi-dimensional (MD) accelerometer data along at least two axes from an accelerometer implanted in the patient during the patient activity. The one or more processors may also be utilized for calculating a travel-related (TR) parameter based on the MD accelerometer data, correlating the TR parameter with physiologic data obtained during the patient activity, and storing the TR parameter and physiologic data as indicators of a current physiologic state of the patient.

Optionally, the initiating, obtaining, calculating, correlating, and storing may be performed at least one of continuously, periodically, in response to the physiologic data satisfying select criteria or in an on-demand basis in response to a clinician request.

Optionally, the initiating may include conveying an initiating instruction from a local external device to an implantable medical device and generating an instruction from the external device for the patient to begin the activity, tracking the data collection interval with a timer; when the timer times-out, conveying a halt instruction from the external device to the IMD and generating a halt instruction from the external device.

Optionally, the method may also include repeating the obtaining and calculating periodically, sorting values for the TR parameter to form an activity histogram indicative of an estimate of patient activity on a periodic basis. In one aspect, the TR parameter may include speed, and the activity histogram represents a period speed histogram, the correlating utilizing a highest speed activity exhibited by the patient during each data collection interval as a basis for the current physiologic state.

Optionally, the method may also include wirelessly communicating between an external device and an implantable medical device (IMD), wherein at least one of the calculating of the TR parameter, the correlation of the TR parameter with the physiologic data, or the storing of TR parameter and physiologic data are implemented at the external device.

Optionally, the patient activity may include implementing a prescribed activity program by a patient. In another aspect, the TR parameter may represent at least one of a distance traveled or a speed, the calculating including calculating TR parameter based on an activity profile histogram.

Optionally, the method may include developing a motion model by obtaining and correlating multiple levels of baseline MD accelerometer data while a patient travels at corresponding baseline speed. In another aspect, the motion model may include activity ranges associated predetermined speeds, the method further comprising identifying sub-intervals of time during which the MD accelerometer data is indicative of a common speed of travel or a speed band having a predetermined speed range, and binning the MD accelerometer data into the activity ranges and based thereon determine speeds traveled for the sub-intervals of time corresponding to segments of the MD accelerometer data.

In one or more embodiment a system for monitoring patient posture may be provided, including an accelerometer configured to be implanted in the patient, the accelerometer configured to generate accelerometer signals along at least first and second axes, the accelerometer signals collectively defining a multidimensional (MD) accelerometer data set. The system may also include a memory configured to store program instructions and to store first and second baseline MD accelerometer data sets associated with first and second reference postures, respectively, and one or more processors. When executing the program instructions, the one or more processors may be configured to obtain, from the accelerometer, a candidate MD accelerometer data set while in a candidate patient posture, the candidate MD accelerometer data set including candidate accelerometer signals along the at least first and second axes, and calculate a candidate - baseline (CB) posture relation. The CB posture relation is calculated between i) the candidate accelerometer signals from the candidate MD accelerometer data set, ii) the accelerometer signals from the first baseline MD accelerometer data set associated with the first reference posture, and iii) the accelerometer signals from the second baseline MD accelerometer data set associated with the second reference posture. The CB posture relation may be indicative of a relation between the candidate patient posture and the first and second reference postures. The one or more processors may also be configured to define and store an actual patient posture based on the CB posture relation.

Optionally, each of the MD accelerometer data sets may include accelerometer signals obtained along X, Y and Z axes that are orthogonal to one another.

Optionally, the CB posture relation may include a first posture relation value indicative of an extent to which the candidate patient posture is common with the first reference posture, and a second posture relation value indicative of an extent to which the candidate patient posture is common with the first reference posture, the one or more processors configured to define the actual patient posture based on the first and second extent values. In one aspect, the first posture relation value is indicative of an extent to which the candidate patient posture corresponds to a vertical posture, and the second posture relation value is indicative of an extent to which the candidate patient posture corresponds to a supine posture. In another aspect, the one or more processors may further be configured to calculate the first and second posture relation values by calculating first and second vector cross products between the accelerometer signals from the candidate MD accelerometer data set and the accelerometer signals from the first and second baseline MD accelerometer data sets.

Optionally, the one or more processors may also be configured to define a threshold map that divides posture relation values into different segments, where each of the segments are indicative of an extent to which a candidate patient posture corresponds to one of the first and second reference postures. In another aspect, the threshold map may include separate segments indicative of an extent to which the candidate patient posture corresponds to at least two of: i) a standing posture, ii) a supine posture, iii) left lateral recumbent posture, iv) right lateral recumbent posture, v) combination of supine and standing postures, vi) combination of supine and left lateral recumbent postures, vii) combination of supine and right lateral recumbent postures, or viii) a recline posture. Optionally, the one or more processors may be further configured to calculate the CB posture relation by calculating: a vector product of at least two vectors of the at least first and second axes.

In one example, the one or more processors may further be configured to obtain, from the accelerometer, the candidate accelerometer signals along the first axis during a first interval, and the candidate accelerometer signals along the second axis during a second interval. In another aspect, the first interval does not overlap with the second interval. Alternatively, the second interval does not begin until the candidate accelerometer signals along the first axis exceed a measurement threshold.

In one or more embodiments, a computer implemented method for monitoring a patient posture may be provide that includes utilizing one or more processors for obtaining, from the accelerometer, a candidate MD accelerometer data set while in a candidate patient posture, the candidate MD accelerometer data set including candidate accelerometer signals along the at least first and second axes, and calculating a candidate - baseline (CB) posture relation. The CB posture relation may be calculated between: i) the candidate accelerometer signals from the candidate MD accelerometer data set, ii) the accelerometer signals from the first baseline MD accelerometer data set associated with the first reference posture, and iii) the accelerometer signals from the second baseline MD accelerometer data set associated with the second reference posture. The CB posture relation may be indicative of a relation between the candidate patient posture and the first and second reference postures. The one or more processors may also be configured to define and store an actual patient posture based on the CB posture relation.

Optionally, each of the MD accelerometer data sets includes accelerometer signals obtained along X, Y and Z axes that are orthogonal to one another.

Optionally, the CB posture relation may include a first posture relation value indicative of an extent to which the candidate patient posture is common with the first reference posture, and a second posture relation value indicative of an extent to which the candidate patient posture is common with the first reference posture, the one or more processors configured to define the actual patient posture based on the first and second extent values. In another aspect, the first posture relation value may be indicative of an extent to which the candidate patient posture corresponds to a vertical posture, and the second posture relation value may be indicative of an extent to which the candidate patient posture corresponds to a supine posture. In yet another aspect, the method may include calculating the first and second posture relation values by calculating first and second vector cross products between the accelerometer signals from the candidate MD accelerometer data set and the accelerometer signals from the first and second baseline MD accelerometer data sets.

Optionally, the method may also include defining a threshold map that divides posture relation values into different segments, where each of the segments are indicative of an extent to which a candidate patient posture corresponds to one of the first and second reference postures. In one aspect, the threshold map includes separate segments indicative of an extent to which the candidate patient posture corresponds to at least two of: i) a standing posture, ii) a supine posture, iii) left lateral recumbent posture, iv) right lateral recumbent posture, v) combination of supine and standing postures, vi) combination of supine and left lateral recumbent postures, vii) combination of supine and right lateral recumbent postures, or viii) a recline posture.

Optionally, the one or more processors may be configured to calculate the CB posture relation by calculating: a vector product of at least two vectors of the at least first and second axes.

Optionally, the method may include obtaining, from the accelerometer, the candidate accelerometer signals along the first axis during a first interval, and the candidate accelerometer signals along the second axis during a second interval. In one aspect, the first interval does not overlap with the second interval. In another aspect, the second interval does not begin until the candidate accelerometer signals along the first axis exceed a measurement threshold.

In one or more embodiments, a system for monitoring real-time activity may be provided. The system may include an accelerometer configured to be implanted in the patient, and the accelerometer may be configured to generate accelerometer signals along three axes. The system may also include a memory configured to store program instructions, and one or more processors. When executing the program instructions, the one or more processors may be configured to obtain, from the accelerometer, accelerometer signals along the three axes while a patient undergoes activity, combine the accelerometer signals along the three axes to form a composite activity signal that is independent of an orientation of the accelerometer, and define at least one of an activity intensity or activity tolerance based on the composite activity signal.

Optionally, the system may also include an implantable medical device (IMD) that houses the accelerometer, a first memory of the memory, and at least a first processor of the one or more processors. The first processor may be configured to obtain the accelerometer signals and form the composite activity signal.

Optionally, each of the composite activity signals may be formed from accelerometer signals obtained along X, Y and Z axes that are orthogonal to one another. In another aspect, the accelerometer signals along the three axes may be obtained sequentially, over a first interval, a second interval, and a third interval, wherein the first interval, the second interval, and the third interval do not overlap. In another aspect, the one or more processors may be further configured to estimate a patient velocity based on the composite activity signal and a piecewise linear function. In another example, the one or more processors may be further configured to determine a patient distance traveled based on the patient velocity determined. Alternatively, the one or more processors may be further configured to determine a number of steps of a patient from the composite activity signal to define the activity intensity or the activity tolerance. In one aspect, the number of steps of the patient may be based on an amplitude or frequency of the composite activity signal.

Optionally, the one or more processors may be further configured to determine step length of a patient or step duration of a patient from the composite activity signal to define the activity intensity of the activity tolerance. In another aspect, the one or more processors are further configured to filter the composite activity signal to remove activity signals having an amplitude below a threshold amplitude.

In one or more embodiments, a computer implemented method for monitoring real-time activity may be provided and include utilizing one or more processors. The one or more processors may be utilized for obtaining, from an accelerometer, accelerometer signals along the three axes while a patient undergoes activity, combining the accelerometer signals along the three axes to form a composite activity signal that is independent of an orientation of the accelerometer, and defining at least one of an activity intensity or activity tolerance based on the composite activity signal.

Optionally, a first processor of the one or more processors obtains the accelerometer signals and forms the composite activity signal.

Optionally, the method may also include forming the composite activity signals from accelerometer signals obtained along X, Y and Z axes that are orthogonal to one another.

Optionally, the method may also include obtaining the accelerometer signals along the three axes sequentially over a first interval, a second interval, and a third interval, wherein the first interval, the second interval, and the third interval do not overlap.

Optionally, the method may also include estimating a patient velocity based on the composite activity signal and a piecewise linear function.

Optionally, the method may also include determining a patient distance traveled based on the patient velocity determined.

Optionally, the method may also include determining a number of steps of a patient from the composite activity signal to define the activity intensity or the activity tolerance. In one aspect, the number of steps of the patient may be based on an amplitude or frequency of the composite activity signal.

Optionally, the method may also include determining step length of a patient or step duration of a patient from the composite activity signal to define the activity intensity of the activity tolerance.

Optionally, the method may also include filtering the composite activity signal to remove activity signals having an amplitude below a threshold amplitude.

Closing

It should be clearly understood that the various arrangements and processes broadly described and illustrated with respect to the Figures, and/or one or more individual components or elements of such arrangements and/or one or more process operations associated of such processes, can be employed independently from or together with one or more other components, elements and/or process operations described and illustrated herein. Accordingly, while various arrangements and processes are broadly contemplated, described and illustrated herein, it should be understood that they are provided merely in illustrative and non-restrictive fashion, and furthermore can be regarded as but mere examples of possible working environments in which one or more arrangements or processes may function or operate.

As will be appreciated by one skilled in the art, various aspects may be embodied as a system, method, or computer (device) program product. Accordingly, aspects may take the form of an entirely hardware embodiment or an embodiment including hardware and software that may all generally be referred to herein as a "circuit," "module" or "system." Furthermore, aspects may take the form of a computer (device) program product embodied in one or more computer (device) readable storage medium(s) having computer (device) readable program code embodied thereon.

Any combination of one or more non-signal computer (device) readable medium(s) may be utilized. The non-signal medium may be a storage medium. A storage medium may be, for example, an electronic, magnetic, optical, electromagnetic, infrared, or semiconductor system, apparatus, or device, or any suitable combination of the foregoing. More specific examples of a storage medium would include the following: a portable computer diskette, a hard disk, a random access memory (RAM), a dynamic random access memory (DRAM), a read-only memory (ROM), an erasable programmable read-only memory (EPROM or Flash memory), a portable compact disc read-only memory (CD-ROM), an optical storage device, a magnetic storage device, or any suitable combination of the foregoing.

Program code for carrying out operations may be written in any combination of one or more programming languages. The program code may execute entirely on a single device, partly on a single device, as a stand-alone software package, partly on single device and partly on another device, or entirely on the other device. In some cases, the devices may be connected through any type of network, including a local area network (LAN) or a wide area network (WAN), or the connection may be made through other devices (for example, through the Internet using an Internet Service Provider) or through a hard wire connection, such as over a USB connection. For example, a server having a first processor, a network interface, and a storage device for storing code may store the program code for carrying out the operations and provide this code through its network interface via a network to a second device having a second processor for execution of the code on the second device.

Aspects are described herein with reference to the figures, which illustrate example methods, devices, and program products according to various example embodiments. The program instructions may be provided to a processor of a general purpose computer, special purpose computer, or other programmable data processing device or information handling device to produce a machine, such that the instructions, which execute via a processor of the device implement the functions/acts specified. The program instructions may also be stored in a device readable medium that can direct a device to function in a particular manner, such that the instructions stored in the device readable medium produce an article of manufacture including instructions which implement the function/act specified. The program instructions may also be loaded onto a device to cause a series of operational steps to be performed on the device to produce a device implemented process such that the instructions which execute on the device provide processes for implementing the functions/acts specified.

The units/modules/applications herein may include any processor-based or microprocessor-based system including systems using microcontrollers, reduced instruction set computers (RISC), application specific integrated circuits (ASICs), field-programmable gate arrays (FPGAs), logic circuits, and any other circuit or processor capable of executing the functions described herein. Additionally, or alternatively, the modules/controllers herein may represent circuit modules that may be implemented as hardware with associated instructions (for example, software stored on a tangible and non-transitory computer readable storage medium, such as a computer hard drive, ROM, RAM, or the like) that perform the operations described herein. The above examples are exemplary only, and are thus not intended to limit in any way the definition and/or meaning of the term "controller." The units/modules/applications herein may execute a set of instructions that are stored in one or more storage elements, in order to process data. The storage elements may also store data or other information as desired or needed. The storage element may be in the form of an information source or a physical memory element within the modules/controllers herein. The set of instructions may include various commands that instruct the modules/applications herein to perform specific operations such as the methods and processes of the various embodiments of the subject matter described herein. The set of instructions may be in the form of a software program. The software may be in various forms such as system software or application software. Further, the software may be in the form of a collection of separate programs or modules, a program module within a larger program or a portion of a program module. The software also may include modular programming in the form of object-oriented programming. The processing of input data by the processing machine may be in response to user commands, or in response to results of previous processing, or in response to a request made by another processing machine.

It is to be understood that the subject matter described herein is not limited in its application to the details of construction and the arrangement of components set forth in the description herein or illustrated in the drawings hereof. The subject matter described herein is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items.

It is to be understood that the above description is intended to be illustrative, and not restrictive. For example, the above-described embodiments (and/or aspects thereof) may be used in combination with each other. In addition, many modifications may be made to adapt a particular situation or material to the teachings herein without departing from its scope. While the dimensions, types of materials and coatings described herein are intended to define various parameters, they are by no means limiting and are illustrative in nature. Many other embodiments will be apparent to those of skill in the art upon reviewing the above description. The scope of the embodiments should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects or order of execution on their acts.

**Claims**

1. A system (100, 1000) for monitoring a physiologic condition of a patient, the system comprising:

   an accelerometer (101, 1170) configured to be implanted in the patient, the accelerometer (101, 1170) configured to collect multi-dimensional (MD) accelerometer data along at least two axes;
   memory (1160) configured to store program instructions;
   one or more processors (1121) that, when executing the program instructions, are configured to:

   initiate a data collection interval in connection with a patient activity;
   collect the MD accelerometer data during the patient activity for at least one of a select period of time or until receiving a data collection halt instruction;
   calculate a travel-related (TR) parameter based on the MD accelerometer data;
   correlate the TR parameter with physiologic data collected during the patient activity; and
   store the TR parameter and the physiologic data as indicators of a current physiologic state of the patient.

2. The system of claim 1, further comprising an implantable medical device (IMD) (100) that houses the accelerometer (101), a first memory (1160) of the memory (1160) and at least a first processor (1121) of the one or more processors (1121), wherein the first processor (1121) is configured to collect the MD accelerometer data.

3. The system of claim 2, wherein the first processor (1121) is configured to implement at least one of the calculating of the TR parameter, the correlation of the TR parameter with the physiologic data, or the storing of TR parameter and physiologic data.

4. The system of claim 2 or 3, wherein the IMD (100) is further configured to wireless transmit, to an external device (1154), at least one of the MD accelerometer data, the TR parameter, or the correlation of the TR parameter and the physiologic data.

5. The system of claim 2, further comprising an external device (1154) configured to wirelessly communicate with the IMD (100), wherein the external device (1154) including a second memory of the memory (1160) and a second processor of the one or more processors (1160), the second processor configured to implement at least one of the calculating of the TR parameter, the correlation of the TR parameter with the physiologic data, or the storing of TR parameter and physiologic data.

6. The system of any one of claims 1 to 5, wherein the patient activity represents a prescribed activity program.

7. The system of any one of claims 1 to 6, wherein the TR parameter represents at least one of a distance traveled or a speed, the one or more processors (1121) configured to calculate the TR parameter based on an activity profile for the corresponding patient.

8. The system of any one of claims 1 to 7, wherein the one or more processors (1121) are further configured to develop a motion model by obtaining and correlating multiple levels of baseline MD accelerometer data while a patient travels at corresponding baseline speeds.

9. The system of claim 8, wherein the motion model includes activity ranges associated predetermined speeds, the one or more processors (1121) further configured to bin the MD accelerometer data into the activity ranges and based thereon determine speeds traveled for sub-intervals of time corresponding to segments of the MD accelerometer data.

10. The system of any one of claims 1 to 9, wherein the calculating includes calculating a speed of movement based on the MD accelerometer data and the motion model.

11. A computer implemented method for monitoring a physiologic condition of a patient, the method comprising: utilizing one or more processors (1121) for,

initiating a data collection interval in connection with a patient activity; obtaining multi-dimensional (MD) accelerometer data along at least two axes from an accelerometer implanted in the patient during the patient activity; calculating a travel-related (TR) parameter based on the MD accelerometer data; correlating the TR parameter with physiologic data obtained during the patient activity; and storing the TR parameter and physiologic data as indicators of a current physiologic state of the patient.

12. The method of claim 11, further comprising repeating the obtaining and calculating periodically, sorting values for the TR parameter to form an activity histogram indicative of an estimate of patient activity on a periodic basis.

13. The method of claim 11 or 12, wherein the TR parameter includes speed and the activity histogram represents a period speed histogram, the correlating utilizing a highest speed activity exhibited by the patient during each data collection interval as a basis for the current physiologic state.

14. The method of any one of claims 1 to 13, further comprising developing a motion model by obtaining and correlating multiple levels of baseline MD accelerometer data while a patient travels at corresponding baseline speeds.

15. The method of claim 14, wherein the motion model includes activity ranges associated predetermined speeds, the method further comprising identifying sub-intervals of time during which the MD accelerometer data is indicative of a common speed of travel or a speed band having a predetermined speed range, and binning the MD accelerometer data into the activity ranges and based thereon determine speeds traveled for the sub-intervals of time corresponding to segments of the MD accelerometer data.

**FIG. 1**

200 ⌐

```
                                                              ⌐202
┌─────────────────────────────────────────────────────────┐
│        Collect Accelerometer Signals Along Two Axes       │
└─────────────────────────────────────────────────────────┘
                          │
                          ▼                                   ⌐204
┌─────────────────────────────────────────────────────────┐
│   Combine Accelerometer Signals to Form Composite Activity Signal │
└─────────────────────────────────────────────────────────┘
                          │
                          ▼                                   ⌐206
┌─────────────────────────────────────────────────────────┐
│    Collect the MD Accelerometer Data During the Patient Activity │
└─────────────────────────────────────────────────────────┘
                          │
                          ▼                                   ⌐208
┌─────────────────────────────────────────────────────────┐
│          Calculate Travel-Related (TR) Parameter          │
└─────────────────────────────────────────────────────────┘
                          │
                          ▼                                   ⌐210
┌─────────────────────────────────────────────────────────┐
│               Calculate Activity Intensity Level          │
└─────────────────────────────────────────────────────────┘
                          │
                          ▼                                   ⌐212
┌─────────────────────────────────────────────────────────┐
│                  Collect Posture Related Data             │
└─────────────────────────────────────────────────────────┘
                          │
                          ▼                                   ⌐214
┌─────────────────────────────────────────────────────────┐
│                  Collect Physiologic Signals              │
└─────────────────────────────────────────────────────────┘
                          │
                          ▼                                   ⌐216
┌─────────────────────────────────────────────────────────┐
│  Assign Risk Factor Threshold for Activity and Physiologic Parameters │
└─────────────────────────────────────────────────────────┘
                          │
                          ▼                                   ⌐218
┌─────────────────────────────────────────────────────────┐
│              Combine the Risk Weighted Parameters         │
│              Into a Risk Weighted Composite Index         │
└─────────────────────────────────────────────────────────┘
                          │
                          ▼                                   ⌐220
┌─────────────────────────────────────────────────────────┐
│                 Determine Physiologic Status             │
└─────────────────────────────────────────────────────────┘
                          │
                          ▼        ⌐222
                    ╱─────────────╲          NO
                   ╱   Composite    ╲ ──────────────┐
                   ╲ Index Exceeds   ╱              │
                    ╲  Threshold?   ╱               │
                     ╲─────────────╱                │
                     YES  │                         │
                          ▼                  ⌐224    │
┌─────────────────────────────────────────────────────────┐│
│   Identify a Treatment Diagnosis and Treatment Notification ││
└─────────────────────────────────────────────────────────┘│
                          │                         │
                          ▼                         │
                     ╭─────────╮                    │
                     │  Done   │◄───────────────────┘
                     ╰─────────╯
```

**FIG. 2**

300

302

Establish Patient
Reference Posture

304

Collect and Record baseline
Accelerometer Signals
along 3D axis

308

Change Ref.
Posture

306

Additional
MD Signals

YES

NO

310

Derive
Synthetic Signal

312

Derive
Synthetic
Signal

YES

NO

Done

FIG. 3A

Standing

**FIG. 3B**

**FIG. 3C**

400

402

Collect an Accelerometer Data Set

404

Obtain a Calibration for the Accelerometer Data Set for Reference Postures

406

Calculate Candidate-Baseline Posture Relations

408

Apply Thresholds to CB Posture Relation Values

410

Define and Store Actual Patient Posture Based on the CB Posture Relation

**FIG. 4A**

**FIG. 3D**

**FIG. 3E**

FIG. 3F

FIG. 3G

**FIG. 3H**

**FIG. 3I**

FIG. 4B

FIG. 4C

FIG. 5A

**FIG. 5B**

**FIG. 6A**

**FIG. 6B**

**FIG. 6C**

**FIG. 7**

EP 4 356 829 A2

## Monitor Physiologic Condition Based on Activity

800

802

Rx Instruction to Begin
Collecting Acceleration Data

804

Collect Acceleration Data
for Select Period of Time
or until instructed to Stop

806

Calculate Travel Related (TR)
Panometer based on Accelerator Data
(Distance max speed, average speed,
speed bins)

808

Correlate TR data with
Physiologic data

810

Store TR data and Physiological data to
Patient Medical Record (PMR) storage

## FIG. 8

**FIG. 9A**

FIG. 9B

**FIG. 10**

**FIG. 11**

EP 4 356 829 A2

**FIG. 12**

**FIG. 13**

FIG. 14

1530

Access Patient Medical Record

1532

Analyze Medication Related Information,
IMD data, BRM data, MD accelerometer data,
profiles etc. based on one or more Application
Specific Model to Assign Health Index
and/or generate a diagnosis

1534

Health Risk
Index Exceed
Threshold?

NO

YES

1536

Generate Treatment Notification
(Clinical Recommendation)

1538

Convey Treatment Notification to
Recipient and/or Device

Done

FIG. 15

## REFERENCES CITED IN THE DESCRIPTION

**Patent documents cited in the description**

- US 20210020294 A
- US 6572557 B
- US 6480733 B
- US 7272443 B
- US 7308309 B
- US 6645153 B
- US 869733
- US 63021778
- US 63139304
- US 9333351 B
- US 9044610 B
- US 9216285 B
- US 8831747 B
- US 8391980 B
- US 9232485 B
- US 10765860 B
- US 10722704 B
- US 20190336747 A
- US 9265428 B
- US 8278941 B
- US 8026729 B
- US 8870787 B
- US 9653926 B
- US 6937900 B
- US 9760679 B